# EUROPÄISCHE PATENTSCHRIFT

(11) **EP 0 595 167 B1**
(45) Veröffentlichungstag und Bekanntmachung des Hinweises auf die Patenterteilung: **07.01.1998**
(21) Anmeldenummer: 93116865.2
(22) Anmeldetag: 19.10.1993
(51) Int. Cl.: C12Q 1/68

(54) **Spezifische Gensonden und Verfahren zur Diagnostik von Candida albicans**
Specific genetic probe and method for candida albicans diagnosis
Sonde génétique spécifique et méthode de diagnostic de Candia albicans

(30) Priorität: 30.10.1992 DE 4236708
(43) Veröffentlichungstag der Anmeldung: 04.05.1994
(73) Patentinhaber: BAYER AG, 51368 Leverkusen (DE)
(72) Erfinder: Springer, Wolfgang, Dr., D-42113 Wuppertal (DE); Plempel, Manfred, Dr., D-42781 Haan (DE); Löbberding, Antonius, Dr., D-42113 Wuppertal (DE)

(56) Entgegenhaltungen:
- NUCLEIC ACID RESEARCH Bd. 19, Nr. 25, 1991, OXFORD Seiten 7113 - 7116 ILIANA OREN ET AL. 'Isolation and Characterisation of a Species-Specific DNA probe for Candida albicans'
- J. CLIN. MICROBIOL. Bd. 30, Nr. 4, April 1992, Seiten 894 - 900 YOZO MIYAKAWA ET AL. 'Isolation and characterization of a species-specific DNA fragment for detection of Candida albicans by PCR'
- J. BACTERIOLOGY Bd. 173, Nr. 2, Januar 1991, Seiten 842 - 850 CHANCHAL SADHU ET AL. 'Telomeric and dispersed repeat sequence in Candida albicans yeast'
- J. CLIN. MICROB. Bd. 26, Nr. 9, September 1988, Seiten 1720 - 1724 J. E. CUTLER ET AL. 'Candida albicans and Candida stellatoidea specific DNA fragment'
- DIAGN. MICROBIOL. INFECT. DIS. Bd. 10, Nr. 3, 1988, Seiten 171 - 179 LORI L. CHEUNG ET AL. 'Development of DNA probes for Candida albicans'

## Beschreibung

Candida ist eine ubiquitäre Hefe, die als Erreger der Candidiasis (Candidamykose) bekannt ist. Mindestens 90 % der Erkrankungen werden verursacht durch die Spezies Candida albicans.

C.albicans ist eine opportunistische Hefe, die in normalen Individuen nur leichte Haut-Infektionen hervorrufen kann. Pilzinfektionen mit schweren Schleimhaut-Infektionen und invasiven Infektionen einzelner Organe werden aber immer häufiger aufgrund der steigenden Zahl an Patienten mit Abwehrschwäche, z.B. Aidspatienten oder immunsuppressiv therapierte Patienten beobachtet.

Unbehandelt führen solche systemischen Infektionen häufig zum Tode der Patienten. Die prinzipiell verfügbare Therapie von invasiven Infektionen basiert z.Zt. auf relativ wenigen Antimykotika wie Amphothericin B, Flucytosin oder die Azolderivate Fluconazol und Itraconazol.

Diese Antimykotika verursachen teilweise verschiedene ernste Nebenwirkungen wie Niereninsuffizienz, Hypokalemie und Anämie ebenso wie unangenehme konstitutionelle Symptome wie Fieber, Schüttelfrost und niedrigen Blutdruck.

Deswegen sind Ärzte und Kliniker daran interessiert für eine direkte und wirksame Therapie über Diagnoseverfahren zu verfügen, die möglichst frühzeitig eine Identifizierung der Pilzerreger ermöglichen.

Herkömmliche Diagnosemethoden basieren auf der in vitro Kultivierung der Erreger und der Identifizierung der Pilzspezies durch morphologische, physiologische und biochemische Methoden. Die Kultivierung von Candida albicans aus Blut ist häufig sehr schlecht und unzuverlässig.

Die Gensonden-Methode insbesondere in Verbindung mit Amplifikationstechniken ist eine schnelle, spezifische und hochempfindliche Methode, die eine Früherkennung der Erreger auf DNA/RNA Ebene ermöglicht. Die Technik kann direkt ohne in vitro Kultivierung im Untersuchungsmaterial durchgeführt werden.

Die DNA-Diagnostik basiert auf der DNA/RNA-Hybridisierungstechnik d.h. der spezifischen in vitro Bindung von komplementärer Einzelstrang-Nukleinsäure unter Bildung von Watson-Crick-Basenpaaren. Die gebildeten DNA/DNA oder DNA/RNA Doppelstränge werden auch als DNA-Hybride bezeichnet. Zur Detektion der spezifischen DNA oder RNA eines Erregers durch die Hybridisierungsreaktion werden komplementäre sequenzspezifische Gensonden verwendet. Diese Gensonden sind entweder kurze, chemisch synthetisierte Oligonukleotidsonden mit einer Länge von 10 bis 100 Nukleotiden oder DNA/RNA Fragmente von 0,5 bis 10 kb, die durch rekombinante Gentechniken hergestellt wurden.

Die Gensonden können photochemisch (N. Dattagupta, et al, Analytical Biochem. 177, 85, 1989) oder enzymatisch durch nick Translation (Rigby, P.W. et al, J. Mol. Biol. 113, 237, 1977) oder Random Primed Techniken (Feinberg und Vogelstein, Anal. Biochem. 132, 6, 1983) mit einer radioaktiven Markierung versehen werden. Geeignet sind hierfür Markierungen mit ³²P NTPs oder nicht radioaktive Markierungen mit Digoxigenin-dUTP, Biotin-dUTP oder direkte Markierung mit Enzymen wie alk. Phosphatase oder Horseradish Peroxidase.

Für die spezifische Hybridisierung zwischen der nachzuweisenden Nukleinsäure des Erregers und der erregerspezifischen Gensonde werden die Nukleinsäuren zunächst durch Denaturierung (Hitze- oder Alkalibehandlung) in Einzelstränge getrennt und dann unter stringenten Bedingungen die durch Temperatur, Ionenstärke der Puffer und organische Lösungsmittel erreicht werden, ganz spezifisch miteinander hybridisiert. Bei geeigneten Hybridisierungsbedingungen bindet die Gensonde nur an komplementäre Sequenzen der nachzuweisenden DNA oder RNA. Diese Hybridisierungsreaktion kann in verschiedenen Testformaten z.B als Festphasenhybridisierung an einen Träger wie z.B. Nitrozellulose gekoppelter Target-DNA oder Gensonde oder als Flüssighybridisierung durchgeführt werden. Die Auswertung (Read Out) erfolgt über die Markierung der Gensonde mit einem Reportermolekül wie oben aufgeführt. Der Hybridisierungskomplex aus Target-DNA und markierter Gensonde wird nach Entfernen von nicht gebundener Gensonde über das verwendete Reportermolekül quantitativ bestimmt. Dieser Read Out kann direkt erfolgen bei Fluoreszenz-Markierung oder radioaktiver Markierung oder indirekt durch Enzymteste und immunologische Verfahren mit Antikörperkonjugaten, die Enzyme wie die alk. Phosphatase enthalten und dann eine Farbreaktion oder Chemilumineszenz-Reaktion ermöglichen.

Die Testsensitivität mit dieser Gensonden-Diagnostik liegt im Bereich 10⁵ bis 10⁶ Keimen auf der Basis der Detektion von Einzelgenen. Eine Erhöhung der Testsensitivität kann durch Kombination mit DNA oder RNA-Amplifikationstechniken wie der PCR (EP 200 362), LCR (EP 320 308), NASBA (EP 329 822), Qβ (PCT 87/06270) oder HAS-Technik (EP 427 074) erreicht werden. Mit diesen Techniken kann bis zu einer 10⁹⁻fachen Multiplikation der nachzuweisenden DNA erzielt werden. Durch die Kombination vom Amplifikation und Hybridisierung wird so die Detektion von einzelnen DNA-Molekülen möglich.

Miyakawa et al. beschreiben im Journal of Clinical Microbiology, Apr. 1992, p. 894-900 die Isolation and Characterization of a Species-Specific DNA Fragment for Detection of Candida albicans by Polymerase Chain Reaction. Diese Literaturstelle enthält keinerlei Hinweise auf die DNA und RNA Fragmente der vorliegenden Erfindung.

Da bei Infektionen im Blut Keimzahlen von 10² bis 10³ Keimen/ml auftreten, bietet sich mit dieser Technologie die Möglichkeit einer frühzeitigen Erkennung von Infektionsverläufen.

Beschrieben werden Gensonden zur Erfassung von Pilzen ganz allgemein zur Differenzierung von Bakterien-, Virus- und Pilzinfektionen (EP 422 872, Genetrak Systems) die auf der Detektion von ribosomalen Sequenzen beruhen. Da mit den heutigen Antimykotika nicht alle Pilzerkrankungen breit therapiert werden können, ist es besonders wichtig, für die einzelnen Erreger geeignete Gensonden zu entwickeln. In der vorliegenden Erfindung werden neue spezifische und besonders sensitive Gensonden für C.albicans beschrieben.

Die neuen Gensonden wurden nach einem Verfahren entwickelt, das spezifische und besonders starke Hybridisierungssignale aufgrund der Hybridisierung mit natürlich vorkommenden amplifizierten Sequenzen liefert. Die Konstruktion von Oligonukleotidsonden und Polynukleotidsonden für C.albicans auf der Basis dieses Verfahrens wird in der Erfindung beschrieben. Die neuen Gensonden basieren nicht auf Gensequenzen von Pathogenitätsfaktoren wie die Aspartatprotease (EP 468 812) oder anderen Strukturgenen (M.M. Mason et al, J.Clin. Microbiol., 25, 563, 1987) (I. Oren et al, Nucleic. Acids Res., 19, 7113, 1991). GenBank und EMBL Nukleotidsequenz-Datenbanken wurden auf Homologie mit unseren Gensondensequenzen überprüft. Es wurden keine Homologien zu bekannten Pilz oder Candida Gensonden gefunden.

Bei den in der Erfindung beschriebenen Gensonden wurde festgestellt, daß sich durch die Kombination von zwei bestimmten Oligonukleotid- oder Polynukleotidsonden das gesamte Spektrum an C.albicans Isolaten erfassen läßt.

Es wird darüber hinaus ein Verfahren zum Einsatz dieser Gensonden in Hybridisierungstesten beschrieben, mit denen eine spezifische Hybridisierung mit Target-DNA von C.albicans erfolgt.

Weiterhin wird in dieser Erfindung die Kombination dieser Hybridisierungsverfahren mit Applikationstechniken beschrieben durch die eine sehr starke Verbesserung der Testsensitivität erzielt wird.

Es wird auch der Einsatz dieser Gensonden und Testverfahren zum Nachweis von C.albicans in klinischem Probematerial beschrieben.

### Konstruktion von spezifischen Gensonden für C.albicans

Zur Entwicklung von spezifischen Gensonden für C.albicans, die eine hohe Testsensitivität aufweisen sollten, wurde nach folgender Strategie vorgegangen:
1. Als Target-Sequenzen sollten Genbereiche ausgewählt werden, die im Genom mehrfach ampliziert vorliegen.
2. Die amplifizierten Sequenzen sollten stark konserviert sein und keine Deletionen oder Insertionen enthalten.
3. Die amplifizierten Sequenzen sollten spezifisch für C.albicans sein und mit keinen anderen Pilz, Bakterien oder anderen eukaryotischen Zellen reagieren.

Zur Identifizierung solcher Gensonden wurde zunächst eine Genbank von C.albicans mit den üblichen gentechnologischen Verfahren (Maniatis et al, Molecular cloning, Cold Spring Harbor Laboratory Press, 1989) in E.coli 5 K hergestellt. Die rekombinanten Klone wurden auf die im Plasmidvektor pBR322 eingebauten C.albicans DNA-Inserte molekularbiologisch analysiert. Klone mit einer C.albicans Insert-Größe von 0,5 bis 7 kb wurden zur weiteren Analyse verwendet.

Durch Reversed Phase Hybridisierung wurde überprüft, welche DNA-Fragmente ein besonders gutes Hybridisierungssignal mit C.albicans DNA ergaben und damit amplifiziert im Genom vorliegen. Dazu wurden die rekombinanten Plasmide aus den E.coli Klonen nach der Southern Blot Technik (Southern, J. Mol. Biol. 98, 503, 1978) gelelektrophoretisch aufgetrennt, auf Nitrozellulosemembranen transferiert und fixiert und dann mit Random Primed markierter genomischer DNA von C.albicans hybridisiert.

Rekombinante Klone, deren C.albicans Inserte besonders starke Hybridisierungssignale aufwiesen, wurden für die weiteren Spezifitätsanalysen eingesetzt.

Die Spezifität der C.albicans Genfragmente wurde durch Slot Blot Tests mit einem Minifold II Filtrationsgerät der Fa. Schleicher und Schüll überprüft. Auf Nitrozellulosemembranen wurde die DNA von verschiedenen Candida-Stämmen, einer Reihe von grampositiven und gramnegativen Bakterien, DNA von anderen Pilzen und verschiedenen Nukleinsäuren von Eukaryontenzellen aufgetragen und fixiert. Die selektionierten C.albicans Genfragmente wurden mit Hilfe der Random Primed Technik mit nicht radioaktiven Reportermolekülen versehen und dann mit den Slot Blots hybridisiert. Gensonden, die nur mit der C.albicans DNA hybridisierten und kein Hybridisierungssignal mit den anderen Nukleinsäurelysaten ergaben, wurden selektioniert.

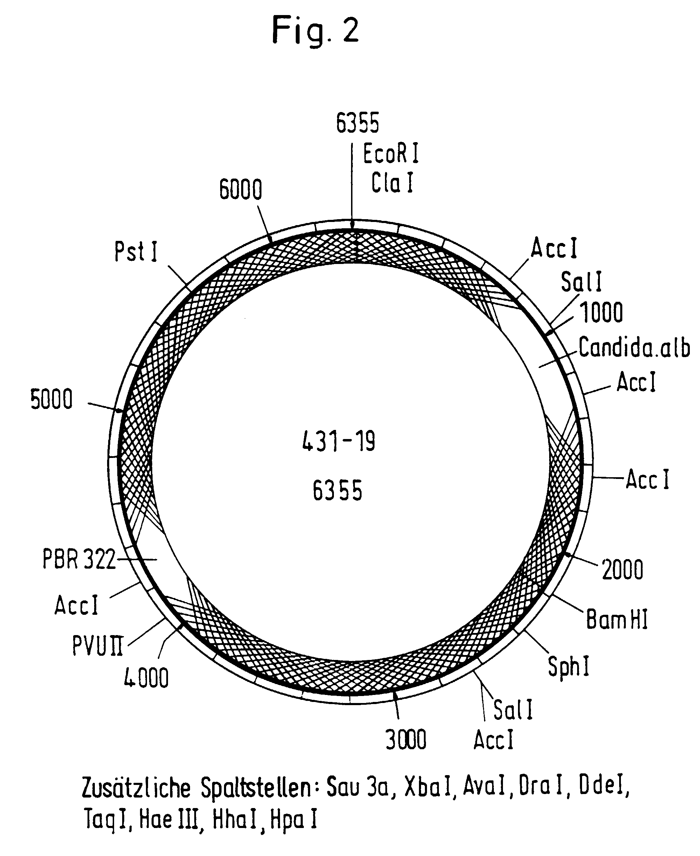

Mit 87 verschiedenen klinischen Isolaten von C.albicans wurde das Erfassungsspektrum der selektionierten spezifischen Gensonden mit der Slot Blot Technik analysiert. Keine der isolierten Gensonden ermöglichte eine gleichzeitige Detektion aller Stämme. Die Gensonde 431-19 mit dem breitesten Erfassungsspektrum für C.albicans ist in Abbildung 2 dargestellt. Mit ihr werden 95 % aller klinischen Isolate erfaßt.

Bei der mikrobiologischen Charakterisierung der nicht detektierten 4 C.albicans Stämme wurde in physiologischen und biochemischen Tests festgestellt, daß es sich um atypische C.albicans-Stämme handelt. Von diesen 4 Stämmen wurden ebenfalls Genbanken hergestellt und wie bei den oben beschriebenen Hybridisierungstesten spezifische Gensonden konstruiert.

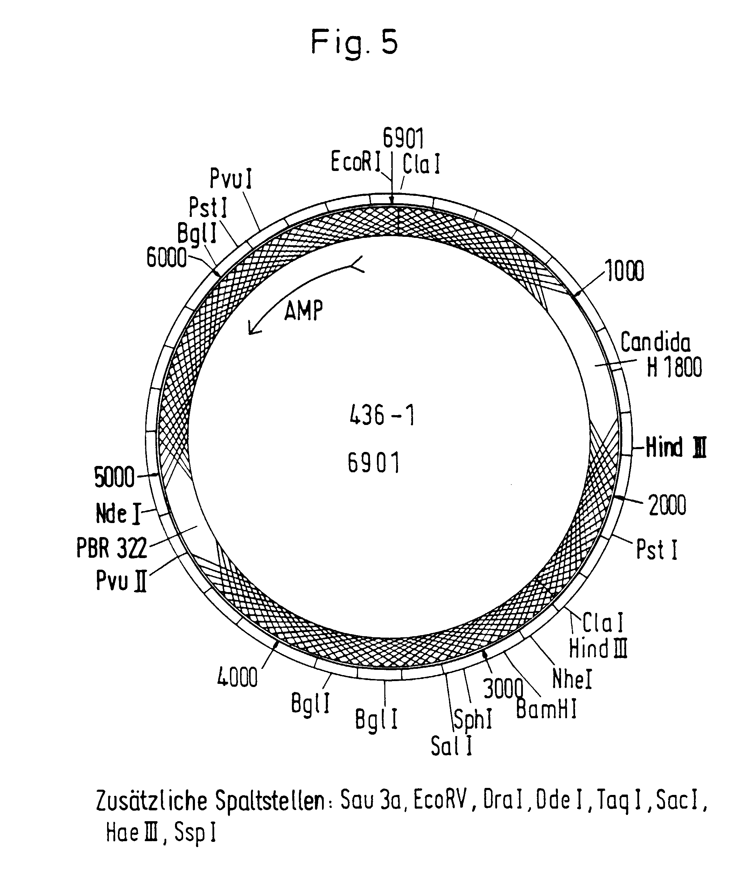

Es wurden Gensonden erhalten, die neben den physiologisch atypischen C.albicans Stämmen auch verschiedene andere C.albicans-Isolate detektierten und Gensonden, die nur die atypischen Isolate nachwiesen. Von der Gensonde 436-1 ist in Abbildung 5 eine Restriktionskarte dargestellt.

Durch die Kombination von Gensonden, wie in den Patentansprüchen definiert, war es möglich, das gesamte Spektrum an C-albicans-Stämmen in einem Hybridisierungstest zu erfassen.

### Molekulare Beschreibung der Polynukleotidsonden

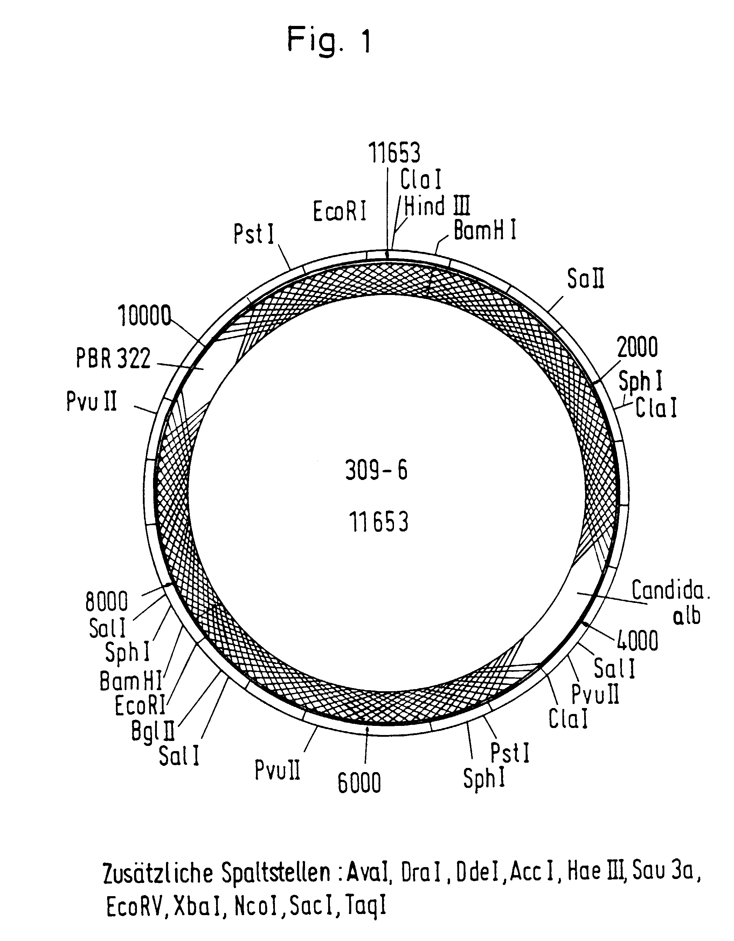

Nach dem oben beschriebenen Verfahren wurden spezifische Polynukleotidsonden für C.albicans konstruiert (Fig. 1-6). Die besten Resultate im Hinblick auf Spezifität, Testsensitivität und Erfassungsspektrum für C.albicans wurden mit der Gensonde 431-19 erreicht. Diese Gensonde wurde aus einer 7 kb Gensonde 309-6 (Fig. 1) durch Subklonierung entwickelt. Dazu wurde die Gensonde zunächst durch Spaltung mit Restriktionsenzymen molekularbiologisch charakterisiert (Fig. 1). Die einzelnen Genfragmente der Gensonde 309-6 wurden in pBR322 Plasmidvektoren als ClaI-BamHI und ClaI-ClaI Fragmente subkloniert und auf ihre Spezifität im Vergleich zur Ausgangssonde analysiert. Für die Gensonde 431-19 wurden die oben beschriebenen Vorteile festgestellt. Eine Restriktionskarte auf der Basis der Restriktionsenzyme ClaI, BamHI, SalI, AccI und SphI ist in Fig. 2 beschrieben. Anhand der vorliegenden Restriktionskarte wurde eine Sequenzierung der Gensonde nach der Methode von Sanger durchgeführt, um auf der Basis der Nukleotidsequenz Oligonukleotidsonden für C.albicans herstellen zu können.

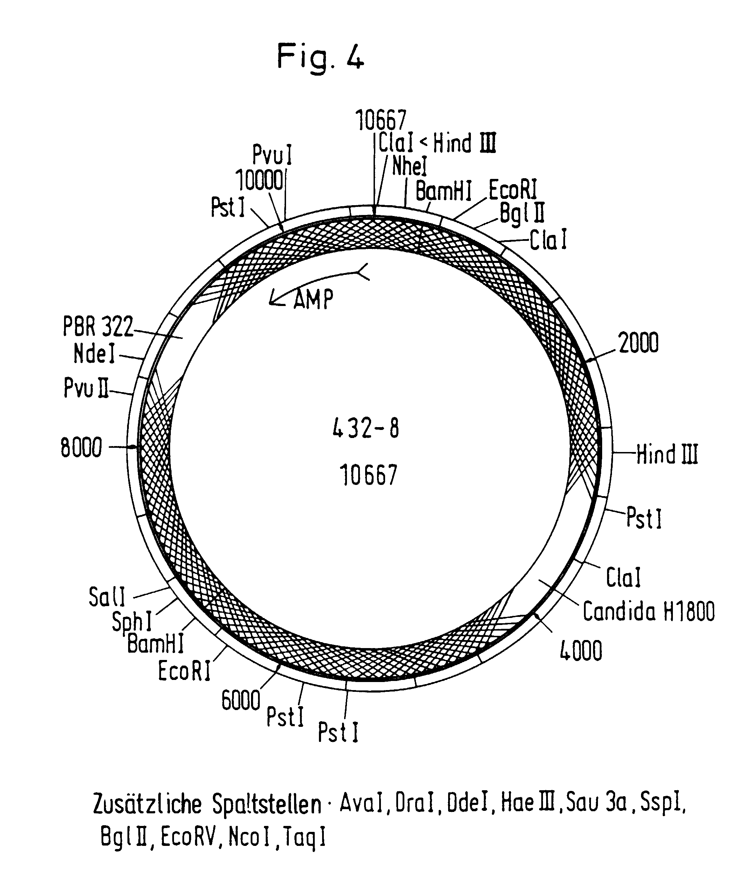

Die Gensonde 436-1, die für die physiologisch atypischen Stämme spezifisch ist, ist in Abbildung 5 beschrieben. Sie wurde aus der 6,3 kb großen Gensonde 432-8 (Fig. 4) durch Subklonierung mit dem Restriktionsenzym ClaI hergestellt. Zur Synthese von Oligonukleotidsonden und zur Anwendung von Amplifikationstechniken wurde ebenfalls eine Sequenzierung der Gensonde vorgenommen.

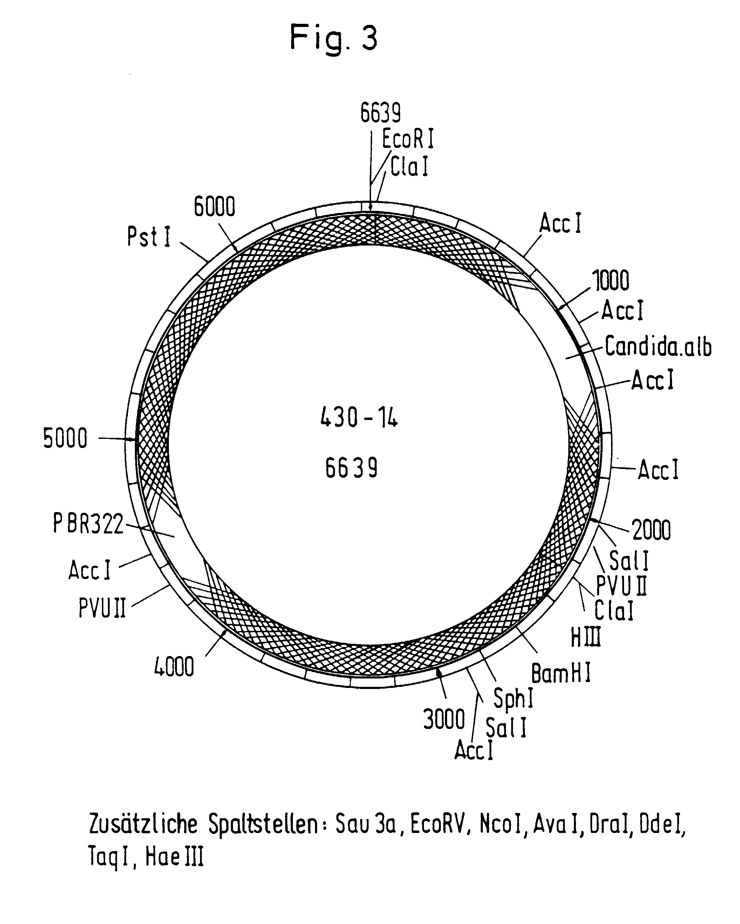

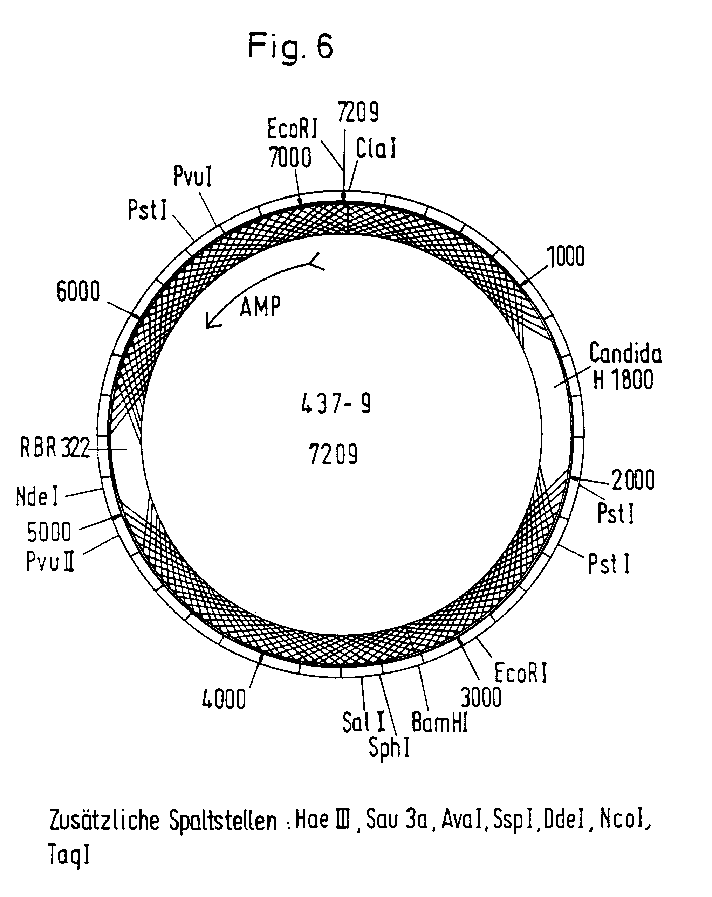

Die Restriktionskarten der Gensonden 430-14 und 437-3 sind in Abbildung 3 und 6 beschrieben. Ihre Hybridisierungseigenschaften gehen aus Tabelle 1 hervor.

### Sequenzierung von Polynukleotid-Sonden

Die Sequenzierung der beschriebenen Gensonden wurde nach der Kettenabbruchmethode von Sanger et al, PNAS, 74, 5463 (1977) mit einem Sequenase-Kit der Firma USB Biochemicals, Ohio, USA durchgeführt. Zur Sequenzierung der Gensonden wurden die Gensonden in den Bluescript Vektor pKS umkloniert und dann mit der Erase a Base Technologie mit einem Kit der Firma Promega Deletionsklone erzeugt. Dieses System basiert auf der Methode von Henikoff S, Gene, 73, 351, 1984, bei der Exonuklease III genutzt wird um DNA spezifisch von überlappenden 5' Enden oder Blunt-Enden zu verdauen. Die gleichmäßige Geschwindigkeit der Degradation durch das Enzym ermöglicht die Erzeugung von Deletionen in vorbestimmten Intervallen durch die zeitabhängige Entnahme von Aliquots aus dem Reaktionsansatz. So können mit dieser Technik in einigen Stunden gleichgerichtete Deletionen über mehrere kbp konstruiert werden.

### Molekulare Beschreibung der Oligonukleotidsonden

Von der Polynukleotidsonde 431.19 wurden 23 100mer Oligonukleotide nach der Phosphoramidit-Methode von S.L.Beaucage und M.H.Caruthers, Tetrahedron Letters 22, 1859, 1981 chemisch hergestellt. Die Sequenzen dieser Oligonukleotidsonden sind wie die komplette Nukleotidsequenz der Gensonde 431-19 im Sequenzprotokoll beschrieben. Die Hybridisierungseigenschaften dieser Oligonukleotidsonden wurde im Vergleich zur Polynukleotidsonde 431-19 an verschiedenen Nukleinsäuren von Candida-Isolaten, grampositiven und gramnegativen Bakterien sowie anderen Pilzen und Eukaryontenzellen ausgetestet (Tabelle 3). Bei den verschiedenen Oligonukleotidsonden wurden teilweise Unterschiede in der Stärke der Hybridisierungssignale festgestellt. In bezug auf die Spezifität für C.albicans wurden jedoch keine signifikanten Unterschiede zwischen den einzelnen Gensonden nachgewiesen. Bei allen 23 Oligonukleotidsonden war eine Kreuzhybridisierung mit Nukleinsäuren von anderen Candida Spezies, Pilzen oder Bakterien nicht zu beobachten. Alle 23 Oligonukleotidsonden eignen sich daher insbesondere in Kombination mit den Oligonukleotidsonden der Polynukleotidsonde 436-1 zum Nachweis von C.albicans.

Von der Polynukleotidsonde 436-1 wurden Oligonukleotidsonden chemisch hergestellt. Die Nukleotidsequenzen der Oligonukleotidsonden sind im Sequenzprotokoll 29369/25-38 dargestellt. Die Hybridisierungseigenschaften dieser Oligonukleotidsonden wurden im Vergleich zur Polynukleotidsonde 436-1 an den vier verschiedenen Nukleinsäurelysaten der atypischen C.albicans-Stämme sowie an grampositiven und gramnegativen Bakterien, Pilzen und Eukaryontenzellen überprüft. Alle 14 untersuchten Oligonukleotidsonden sind spezifisch für die 4 seltenen C.albicans Stämme, unterscheiden sich jedoch teilweise in ihrer Hybridisierstärke mit den verschiedenen Candida-Stämmen.

Oligonukleotidsonden der Polynukleotidsonde 431-19 und der Polynukleotidsonde 436-1 konnten beliebig kombiniert werden um alle C.albicans Isolate in einem Hybridisierungstest zu erfassen.

### Beispiel 1

### C.albicans Nachweis durch Slot Blot Hybridisierung mit der Polvnukleotidsonde SPN 431.19

Im Rahmen der Slot Blot Hybridisierung wurde die Nukleinsäure aus dem Probenmaterial durch an sich bekannte analytische oder präparative Nukleinsäureverfahren isoliert und dann auf Membranen wie Nitrozellulose oder Nylon fixiert, die in verschiedenen Ausführungen kommerziell erhältlich sind. Die Nukleinsäure kann mit einem Filtrationsgerät (z.B. Schleicher und Schüll Minifold II) auf die Membranen aufgetragen und fixiert werden und dann mit der mit Reportermolekülen versehenen Gensonden unter geeigneten Stringenzbedingungen auf Hybridisierung getestet werden. Das Hybridisierungssignal ist umso besser, je höher die Komplementarität der Nukleotidsequenzen von Target-DNA und Gensonden-DNA ist. Der Read Out der Slot Blots oder Dot Blots erfolgt je nach Markierung der Gensonde z.B. durch Autoradiographie (³²P), Farbsignal (alk. Phosphatase, Bromchlorindolylphosphat/Nitroblau-Tetrazolium) oder Chemilumineszenz sowie Fluoreszenz.

### Isolierung von Candida Nukleinsäure

Die im YM-Medium (Bacto-Yeast Extrakt 4g; Bacto Malzextrakt 10 g; Bacto-Dextrose 4 g; H₂O 1l, pH 7,3) angezüchteten Candida-Stämme wurden 5 Minuten bei 5000 UpM abzentrifugiert und 2 mal mit 1 M Sorbitol gewaschen.

Die Zellen wurden in 1 ml Sorbitol mit 100 µg Zymolase-100T aus Arthrobacter luteus (10 000 Units/g) resuspendiert und ca. 30 Minuten bei 37°C inkubiert.

Die Proben wurden anschließend mit 200µl EDTA 0,5M pH8,0 und 200µl 10 %igem Lauroylsarcosin versetzt und im Wasserbad bei 100°C 3 Minuten erhitzt. Nach dem Abkühlen in Eis/Isopropanol wurden die Proben mit 1x TE (10mM Tris/HCl pH 8,1mM EDTA) auf 20 ml aufgefüllt und 2x mit Tris gesättigtem Phenol behandelt, bei 6000 UpM zentrifugiert und die wäßrige DNA-Phase 2x mit Ether extrahiert um Phenolreste zu entfernen. Nach dem Ausfällen der DNA mit Isopropanol und Waschen des DNA-Pellets mit 70 % Ethanol wurden die Proben in 1-2 ml 1x TE aufgenommen und durch Elektrophorese in 0,8 %igen Agarosegel analysiert.

### Isolierung von Bakterien-Nukleinsäure

Für Dot Blot Hybridisierungen wurden 1,5 ml Bakterienkulturen, bei präparativen Isolierungen 10 x 1,5 ml Bakterienkultur in Eppendorfansätzen oder 150 ml mit entsprechend größeren Pufferansätzen eingesetzt.

Die Methode wird für 1,5 ml Bakterienkulturen beschrieben. 1,5 ml Bakterienkulturen werden bei 6000 UpM abzentrifugiert. Das Pellet wird mit 500 µl 15 %iger Saccharose in TE+ 2 mg/ml Lysozym versetzt. Nach 30 Minuten Inkubation bei 37°C werden 50 mi 0,5 M EDTA pH 8 zugegeben und bei schlechter Lyse 10 Minuten bei 65°C behandelt bis eine vollständige Lyse eintritt.

Zur weiteren Aufreinigung werden 1 Volumen Phenol zugegeben, gut gemischt und dann 10 Minuten bei 10 000 UpM zentrifugiert. Die obere DNA-haltige Phase wird mit 100 µl Diethylether (Tris gesättigt) gemischt und dann 1 Minute bei 10 000 UpM zentrifügiert. Die obere Etherphase wird verworfen. Die untere DNA Phase wird mit 1/10 Volumen 3M Natriumacetat und 1 Volumen Isopropanol versetzt und 5 Minuten bei Raumtemperatur stehen gelassen. Anschließend wird 10 Minuten bei 10 000 UpM zentrifugiert, der Überstand dekantiert und das Pellet im halben Ausgangsvolumen in TE gelöst.

### Isolierung und Markierung der Gensonde SPN 431.19

Die Markierung der Polynukleotidsonden wurde mit der Random primed Methode nach Feinberg und Vogelstein (Anal. Biochem. 132, 6, 1983) oder durch 3'Endgruppenmarkierung mit terminaler Transferase (Chang, L.M.S., Bollum T.J., J. Biol. Chem. 246, 909, 1971) durchgeführt.

### Durchführung der Slot Blot Hybridisierung mit der Gensonde SPN 431.19

Die Nukleinsäure wird mit TE auf ein Volumen von 200 µl eingestellt. Anschließend wird die DNA 10 Minuten bei 100°C denaturiert und dann die Ansätze sofort in Eis/NaCl Gemisch gestellt. 200 µl eiskaltes 20x SSC werden zugegeben und sofort auf Nitrozellulose/Nylon-Filter mit Hilfe eines Minifold II-Filtrationsgerätes der Firma Schleicher und Schüll aufgetragen.

Die Filter wurden vorher 10 Minuten in dest. H₂O und dann 10 Minuten in 10x SSC vorbehandelt und nach dem DNA-Auftrag 2 Stunden bei 80°C gebacken.

Festphasen-Hybridisierungen mit Nitrozellulose oder Nylonmembranen wurden nach der Slot Blot Methode durchgeführt.

Der Read Out erfolgte mit Antidigoxigenin-Antikörper-Konjugaten mit alkalischer Phosphatase und Brom-Chlor-Indolylphosphat/Nitroblautetrazolium als Farbstoffreaktion oder mit Adamantan-Dioxetan (AMPPD) als Chemilumineszenz-Reaktion.

Für die Hybridisierungsexperimente wurde die DNA zunächst durch 10 minütiges Erhitzen auf 100°C und schnellem Abkühlen auf Eis/NaCl in die DNA-Einzelstränge denaturiert. Nitrozellulosemembranen wurden durch Quellen in Wasser und 20 x SSC (NaCl 3 Mol/l, Nacitrat 0,3 Mol/l pH 7) vorbehandelt und anschließend getrocknet. Nylonmembranen wurden ohne Vorbehandlung eingesetzt. Die denaturierten Nukleinsäureextrakte wurden mit einem Filtrationsgerät auf die Nitrozellulose oder Nylonmembranen aufgetragen und anschließend durch 1 Stunde Backen bei 80°C im Vakuum oder durch 5 Minuten UV-Vernetzung mit einem UV-Transilluminator (Nylonmembran) fixiert. Die Nylon/Nitrozellulose-Filter wurden in einem Plastikbeutel mit 20 ml Hybridisierungs-Lösung (5xSSC; Blocking Reagenz 0,5 %, N-Lauroylsarkosin.Na-Salz 0,1 %, SDS 0,02 %) verschweißt und 1 Stunde bei 68°C prähybridisiert. Die Prähybridisierungslösung wurde dann durch 2,5 ml Hybridisierungslösung (gleiche Zusammensetzung) ersetzt, die frisch denaturierte Gensonden - DNA (100 ng) enthielt. Der Hybridisierungsansatz wurde 2 Stunden bei 68°C inkubiert. Die Filter wurden anschließend 2x5 Minuten bei Raumtemperatur mit jeweils 50 ml 2 x SSC, SDS 0,1 % und dann nochmals 2 x 15 Minuten bei 68°C mit 0,1 x SSC, 0,1% SDS gewaschen. Die Filter wurden für die Detektion der hybridisierten DNA direkt verwendet oder luftgetrocknet für den späteren Nachweis aufbewahrt.

### Detektion der hybridisierten DNA

Zur quantitativen Detektion der hybridisierten C. albicans-DNA wurde eine immunologische Nachweisreaktion durchgeführt. Verwendet wurde ein Antikörperkonjugat mit gekoppelter alk. Phosphatase, das an die hybridisierte Digoxigenin-markierte DNA bindet. Die Farbreaktion wurde bei alkalischem pH durch Zugabe des farblosen 5-Brom-4-chlor-3-indolylphosphat und Nitroblautetrazolium nach 2 bis 12 Stunden quantitativ mit einem Densitometer der Firma Shimadzu CS-430 ausgewertet.

Für die Nachweisreaktion wurden folgende Puffer eingesetzt:

| | |
|---|---|
| Puffer 1 | Tris/HCl 100 mmol/l pH 7,5 |
| Puffer 2 | Blocking Reagens 0,5 % in Puffer 1 lösen |
| Puffer 3 | Tris/HCl 100 mmol/l, MgCl₂ 50 mmol/l pH 9,5 |
| Puffer 4 | Tris/HCl 10 mmol/l, EDTA 1 mmol/l pH 8 |

Farblösung (frisch zubereitet) 45 µl NBT und 35 µl X-Phosphat wurden zu 10 ml Puffer 3 zugegeben.

Die Nitrozellulose/Nylonfilter wurden 1 min in Puffer 1 gewaschen und dann 30 min mit 100µl Puffer 2 inkubiert und nochmals mit Puffer 1 gewaschen. Das Antikörperkonjugat wurde 1:5000 in Puffer 1 verdünnt und die Filter 30 min mit ca. 20µl der verdünnten Antikörper-Konjugatlösung inkubiert. Ungebundenes Antikörper-Konjugat wurde durch 2 x 15 min Waschen mit 100 µl Puffer 1 entfernt und die Filter anschließend 2 min mit 20 µl Puffer 3 äquilibiert. Die Filter wurden dann mit 20ml Farblösung in einem verschlossenen Plastikbeutel im Dunkeln inkubiert. Die Farbintensität der einzelnen Slot Blots wurde im Vergleich zu einem mit aufgetragenen C. albicans-DNA-Standard densitometrisch bestimmt.

### Spezifität der C. albicans Gensonde 431-19

In der Tabelle 1 sind die Hybridisierungseigenschaften der Gensonde 431-19 mit Nukleinsäuren aus verschiedenen Candida-Stämmen, gramnegativen und grampositiven Bakterien, verschiedenen anderen Pilzen und Eukaryontenzellen mit dem obigen Hybridisierungstest zusammengefaßt. Die Hybridisierungsdaten zeigen, daß die Gensonde absolut spezifisch für Candida albicans ist. Andere Nukleinsäuren werden durch diese Gensonde nicht erfaßt.

### Detektionsspektrum der Gensonde 431.19 für C. albicans Isolate

In der Tabelle 2 sind die Hybridisierungseigenschaften der Gensonde 431.19 mit Nukleinsäuren aus 87 C. albicans Isolaten zusammengefaßt. Die Hybridisierungsdaten zeigen, daß die Gensonde mit 83 C. albicans Stämmen hybridisiert, 4 Isolate werden dagegen nicht erfaßt. Diese 4 Stämme zeigen in physiologischen und biochemischen Tests, daß es sich um keine typischen C. albicans Isolate handelt. Die C. albicans Zugehörigkeit wird durch diese Tests jedoch bestätigt.

### Beispiel 2

### C. albicans Nachweis durch Slot Blot Hybridisierung mit der Gensonde 436.1

Die Isolierung der Nukleinsäuren aus den verschiedenen Testproben, die Isolierung und Markierung der Gensonde und die Slot Blot Hybridisierung wurden wie im Beispiel 1 durchgeführt.

### Spezifität der C. albicans Gensonde 436.1

In der Tabelle 1 sind die Hybridisierungseigenschaften der Gensonde 436.1 mit Nukleinsäuren aus verschiedenen Candida-Stämmen, grampositiven und gramnegativen Bakterien, verschiedenen anderen Pilzen und Eukaryontenzellen mit dem im Beispiel 1 beschriebenen Slot Blot Test zusammengefaßt. Die Hybridisierungsdaten zeigen, daß die Gensonde spezifisch für die 4 atypischen Candida-Stämme ist, während andere Candida albicans Stämme oder andere Candida Spezies andere Bakterien, Pilze und Eukaryontenzellen nicht detektiert werden.

### Detektionsspektrum der Gensonde 436.1 für C. albicans Isolate

In der Tabelle 2 sind die Hybridisierungseigenschaften der Gensonde 436.1 mit Nukleinsäuren aus 87 C. albicans Isolate zusammengefaßt. Die Hybridisierungsdaten zeigen, daß die Gensonde mit den 4 seltenen C. albicans Stämmen hybridisiert, die 84 anderen C. albicans Stämme werden jedoch nicht erfaßt.

### Beispiel 3

### C.albicans Nachweis durch Slot Blot Hybridisierung mit der GensondenKombination 431.19 und 436.1

Die Isolierung der Nukleinsäure aus dem Probenmaterial, die Isolierung und Markierung der Gensonden und die Slot Blot Hybridisierung wurden wie im Beispiel 1 und 2 durchgeführt. Es wurden jeweils 100 ng der beiden Gensonden für das Hybridisierungsexperiment eingesetzt.

### Spezifität der Gensondenkombination 431.19 und 436.1

In der Tabelle 1 sind die Hybridisierungseigenschaften der kombinierten Gensonden 431.19 und 436.1 mit Nukleinsäuren aus verschiedenen Candida-Isolaten, gramnegativen und grampositiven Bakterien, anderen Pilzen und Eukaryontenzellen mit dem Slot Blot Test zusammengefaßt.

Die Hybridisierungsdaten zeigen, daß mit der Gensondenkombination spezifisch C. albicans Stämme detektiert werden und keine Hybridisierung mit anderen Candida Spezies oder anderen Pilzen und Bakterien erfolgt.

### Detektionsspektrum der kombinierten Gensonden 431.19 und 436.1 für C. albicans Isolate

In der Tabelle 2 sind die Hybridisierungseigenschaften der kombinierten Gensonden 431.19 und 436.1 mit Nukleinsäuren aus 87 C. albicans Isolaten zusammengefaßt. Die Hybridisierungsdaten zeigen, daß die kombinierten Gensonden mit allen 87 C. albicans Stämmen hybridisieren.

### Beispiel 4

### Nachweis von C. albicans durch Slot Blot Hybridisierung mit Oligonukleotidsonden

Im Sequenzprotokoll sind die Sequenzen der Oligonukleotidsonden auf der Basis der Polynukleotidsonde 431.19 (29369/2-24) und die Oligonukleotidsonden auf der Basis der Polynukleotidsonde 436.1 (29369/25-38) dargestellt. Die Synthese dieser Oligonukleotidsonden erfolgte nach der Phosphoramidit-Methode von C.L. Beaucage und M.H. Caruthers, Tetrahedron Letters 22, 1859 (1981).

Die Markierung der Oligonukleotidsonden wurde mit einem 3' oder 5' Endgruppenmarkierungskit der Firma Boehringer, Mannheim mit terminaler Transferase bzw. Polynukleotidkinase durchgeführt. Als Reportermolekül wurden alpha³²PdCTP oder gamma³²PATP oder nicht radioaktives dUTP-Digoxigenin für die 3' Endmarkierung eingesetzt.

Die Slot Blot Hybridisierung wurde wie im Beispiel 1 durchgeführt. In der Tabelle 3 ist die Hybridisierung der Oligonukleotidsonden 2-6 und 25-27 mit Nukleinsäuren aus verschiedenen Candida Spezies, grampositiven und gramnegativen Bakterien, anderen Pilzen und Eukaryontenzellen beispielhaft für alle getesteten Oligonukleotidsonden aufgeführt.

Die Hybridisierungsdaten ergeben, daß die in der Tabelle 3 aufgeführten Oligonukleotidsonden 2-6 wie auch die ebenfalls getesteten in der Tabelle nicht aufgeführten Oligonukleotidsonden 7-24 spezifisch mit C. albicans hybridisieren und kein Signal mit anderen Candida Spezies oder anderen Bakterien und Pilzen ergeben. Lediglich Unterschiede in der Hybridisierungsstärke wurden bei den einzelnen Oligonukleotidsonden festgestellt.

Die Hybridisierungsexperimente wurden auch mit kürzeren Oligonukleotidsonden (bis 15mere) mit dem gleichen Hybridisierungsergebnis durchgeführt.

Die Oligonukleotidsonden 25-38 auf der Basis der Polynukleotidsonde 436.1 hybridisierten wie die Polynukleotidsonde nur mit den seltenen 4 C. albicans Stämmen.

Eine beliebige Kombination von Oligonukleotidsonden der 431.19 Serie mit Oligonukleotidsonden der 436.1 Serie ermöglichte dagegen die Detektion aller 87 C. albicans Stämme in einem Hybridisierungsexperiment.

### Beispiel 5

### Nachweis von amplifizierter C. albicans DNA durch Flüssighybridisierung mit den kombinierten Gensonden 431.19 und 436.1

Durch die Kombination von geeigneten Target-Amplifkationsmethoden wie PCR (EP 200 362), LCR (EP 320 308), NASBA (EP 329 822), Qβ (PCT 87/06270) oder HAS (EP 427 074) und der Gensondentechnik wird eine signifikante Sensitivitätssteigerung gegenüber den herkömmlichen Gensonden-Read Out Methoden erzielt.

Die Amplifikation der Target-DNA wurde nach der Polymerase Chain Reaktion und alternativ nach der Hairpin Amplifikationsmethode (HAS) durchgeführt.

Zur PCR-Reaktion wurden eingesetzt 1 bis 1000 pg genomische DNA von C. albicans, 2 µmol Primer 1 (5' dTCGTCGATGGTATCGTCGTTCTGC) (SEQ ID NO 39) oder bevorzugt Primer 1'(5'dCCGAACCGCCATACCAGAAGCATT) (SEQ ID NO 40) und Primer 2 (5'dCCGAACCGCGATACCAGAAGCATT) (SEQ ID NO 41), 2,5 Units Taq-Polymerase von Cetus/Perkin-Elmer sowie jeweils 200 µmol dNTPS und 6 µmol Digoxigenin und UTP in insgesamt 100 µl PCR-Puffer (50 mM KCl, 10 mM Tris/HCl pH pH 8,3, 1,5 mM MgCl₂ und 0,01 % Gelatine). Zur Amplifikation der genomischen DNA von atypischen C.albicans-Stämmen (436.1 Gensonde) wurden zusätzlich die Primer 3 (5'dTTCTTACGAATGTTTGTGTT) (SEQ ID NO 42) oder bevorzugt Primer 3'(5'dATCCACCAATATATATACACA) (SEQ ID NO 43) und Primer 4(5'dTTTGTCCTATTGGTACAGTT) (SEQ ID NO 44) zugesetzt. Die Amplifikation wurde in einem PCR-Prozessor der Firma Cetus/Perkin-Elmer durchgeführt.

Mit den Ansätzen wurde zunächst eine Initialschmelzung der DNA 2 Minuten, 30 Sekunden bei 94°C durchgeführt, dann pro Zyklus 1 Minute bei 94°C die DNA denaturiert, 2 Minuten bei 40 bis 45°C das Primer-Annealing und 3 Minuten bei 72°C die Primer-Extension durchgeführt. Nach 35 Zyklen wurde abschließend eine 20 Minuten-Extension bei 72°C durchgeführt und die Ansätze bei 4°C gekühlt

Die Flüssighybridisierungsteste wurden als Reversed Phase Teste mit 100 ng 5'-biotinylierten Gensonden und amplifizierter mit Digd UTP markierter DNA von C. albicans in einem Volumen von 50 µl durchgeführt.

Nach 10 minütigem Erhitzen auf 100°C und anschließendem Abkühlen auf 0°C wurden 50 µl 2 x Boehringer Hybridisierungsmix zugegeben und 1 Stunde bei 68°C hybridisiert Zur Separation des Hybridisierungskomplexes durchgeführt wurden 75µl Streptavidin gecoatete magnetische Partikel der Firma Dynal eingesetzt. Die magnetischen Beads wurden mit 1 x Boehringermix vorbehandelt und nach dem Separieren über einen Magneten die Flüssigkeit abpipettiert und der Hybridisierungsansatz zugegeben und 1/2 Stunde bei Raumtemperatur unter schwacher Bewegung inkubiert. Der gekoppelte Hybridisierungskomplex wurde mit den Beads separiert, die Restflüssigkeit abpipettiert und einmal mit Puffer A (2 x SSC; 0,1 % SDS), dann mit Puffer B (0,1 SSC; 0,1 % SDS) 2 x gewaschen.

Anschließend wurde die Blocking-Reaktion und die Antikörper-Reaktion zum Nachweis der Hybridisierung über Chemilumineszenz durchgeführt. Die mit DNA beladenen Beads wurden 1 x mit 150 µl Waschpuffer (0,1 M Maleinsäure, 0,1 M NaCl, pH 5, 0,3 % Tween 20) behandelt und nach dem Separieren und Abpipettieren des Waschpuffers 400 µl Puffer 2 (0,1 M Maleinsäure; 0,15 M NaCl, pH 7,5; 1 % Blocking Reagenz (Boehringer)) zugegeben. Nach 1/2 Stunde Inkubation bei Raumtemperatur wurde separiert, abpipettiert und 100 µl Antikörperkonjugat-Lösung (AK 1:10 000 in Puffer 2) zugegeben und 1/2 Stunde bei Raumtemperatur inkubiert, dann separiert, abpipettiert und mit 400 µl Waschpuffer behandelt 2 x 15 Minuten bei schwacher Bewegung. Anschließend wurde separiert und mit 150 µl Puffer 3 (0,1 M Tris/HCl Puffer mit 0,1 M NaCl und 50 mM MgCl₂ pH 9,5) behandelt. Es wurde wieder separiert und mit 100 µl Detektionslösung mit AmPPD 1:100 in Puffer 3 15 Minuten bei 37°C im Wasserbad inkubiert, dann die Chemilumineszenz im Lumineszenz-Photometer bei 477 nm (Lumacounter von Lumac) gemessen. Mit diesem Test können DNA-Mengen entsprechend 10⁶ bis 10 C.albicans Keime detektiert werden. Die gleiche Detektionsgrenze von 10 Keimen pro ml Blut wurde auch nach Zugabe von unspezifischer Blut-DNA erreicht. Blut-DNA alleine gibt nur geringe Background-Signale im Hybridisierungstest.

### Beispiel 6

### Nachweis einer Candidamykose im Blut, Sputum oder Biopsiematerial durch Amplifikation und Hybridisierung mit den Gensonden 431.19 und 436.1

Das klinische Probenmaterial wurde durch geeignete mechanische oder chemische Aufschlußmethoden wie z.B. mit Detergentien wie SDS homogenisiert und lysiert. Im Fall von Blut als Ausgangsmaterial wurden die Proben direkt mit sterilem Wasser lysiert und das abzentrifugierte Blutpellet mit den C. albicans Keimen dann nach der im Beispiel 1 beschriebenen enzymatischen Aufschlußmethode lysiert. Das C. albicans/Blut-Lysat wurde dann mit Hilfe geeigneter Amplifikationsmethoden wie im Beispiel 5 beschrieben mit C. albicans spezifischen Oligonukleotid-Primern amplifiziert. Die amplifizierte Nukleinsäure wurde dann mit den Polynukleotidsonden 431.19 und 436.1 hybridisiert und der unter stringenten Bedingungen sich ausbildende spezifische Hybridisierungskomplex aus amplifizierter C. albicans Nukleinsäure und Gensonden-DNA wurde mit magnetischen Partikeln der Firma Dynal separiert und wie im Beispiel 5 durch Chemilumineszenz-Read Out quantitativ bestimmt. Der Test kann auch mit den im Sequenzprotokoll aufgeführten Oligonukleotidsonden durchgeführt werden.

In den mit C. albicans infizierten Blutlysaten wurden C. albicans spezifische Hybridisierungssignale noch in einer Konzentration von 10 Keimen problemlos nachgewiesen.

### Kurze Beschreibung der Abbildungen:

- Fig. 1:: Restriktionskarte des 7,2 kb BamHI Gensondenfragmentes 309-6 kloniert in den Plasmidvektor pBR 322. Weitere Restriktionsenzyme, die die Gensonde schneiden/nicht schneiden sind im Text aufgeführt.
Die Gensonde stammt aus dem Stamm C. albicans Hantschke
- Fig. 2:: Restriktionskarte des 2,2 kb ClaI-BamHI Gensondenfragmentes 431-19 kloniert in den Plasmidvektor pBR 322. Weitere Restriktionsenzyme, die die Gensonde schneiden/nicht schneiden sind im Text aufgeführt.
Die Gensonde stammt aus dem Stamm C. albicans Hantschke.
- Fig. 3:: Restriktionskarte des 2,3 kb ClaI-ClaI Gensondenfragmentes 430-14 kloniert in den Plasmidvektor pBR 322. Weitere Restriktionsenzyme, die die Gensonde schneiden/nicht schneiden sind im Text aufgeführt.
Die Gensonde stammt aus dem Stamm C. albicans Hantschke.
- Fig. 4:: Restriktionskarte des 6,3 kb BamHI Gensondenfragmentes 432-8 kloniert in den Plasmidvektor pBR 322. Weitere Restriktionsenzyme, die die Gensonde schneiden/nicht schneiden sind im Text aufgeführt.
Die Gensonde stammt aus dem Stamm C. albicans H 1800.
- Fig. 5:: Restriktionskarte des 2,6 kb ClaI Gensondenfragmentes 436-1 kloniert in den Plasmidvektor pBR 322. Weitere Restriktionsenzyme, die die Gensonde schneiden/nicht schneiden sind im Text aufgeführt.
Die Gensonde stammt aus dem Stamm C. albicans H 1800.
- Fig. 6:: Restriktionskarte des 3,2 kb ClaI-BamHI Gensondenfragmentes 437-3 kloniert in den Plasmidvektor pBR 322. Weitere Restriktionsenzyme, die die Gensonde schneiden/nicht schneiden sind im Text aufgeführt.
Die Gensonde stammt aus dem Stamm C. albicans H 1800.

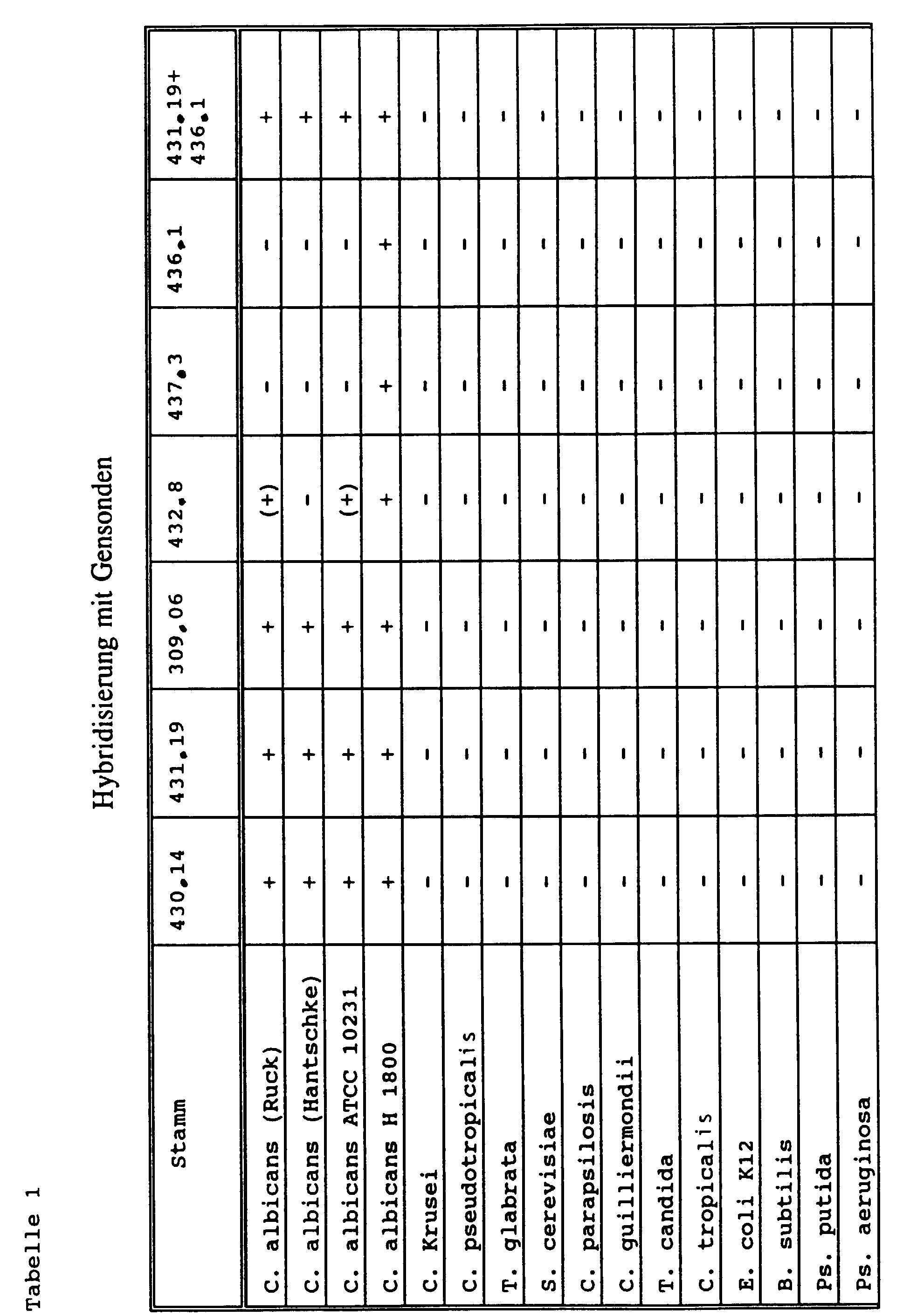

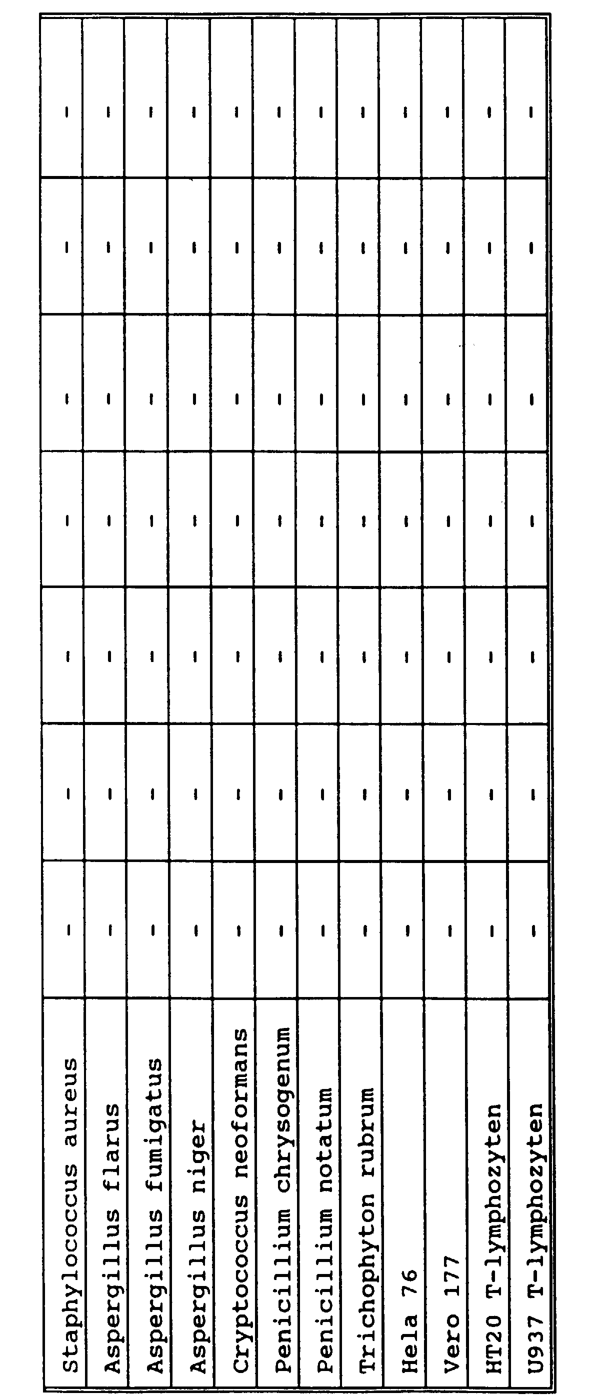

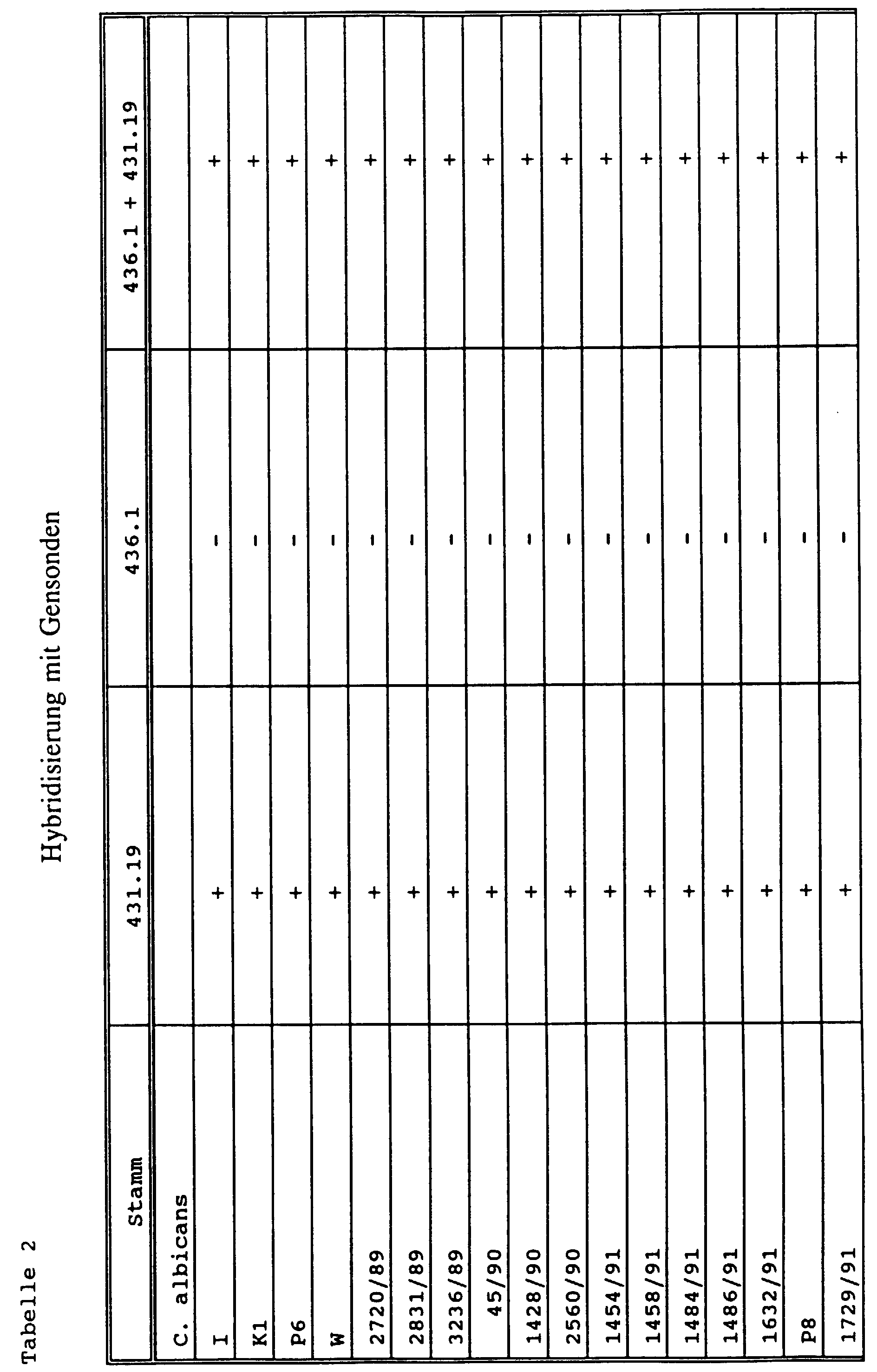

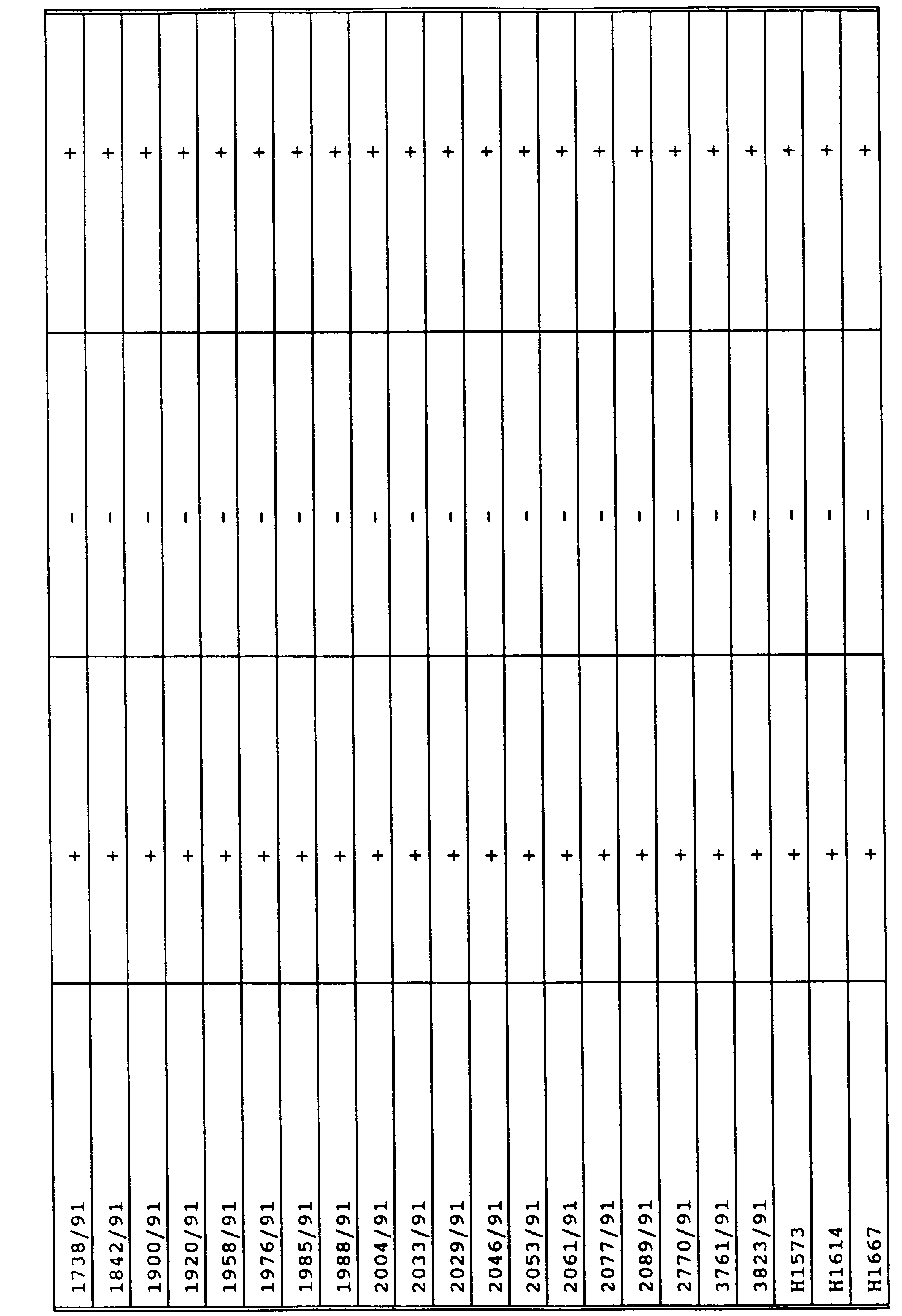

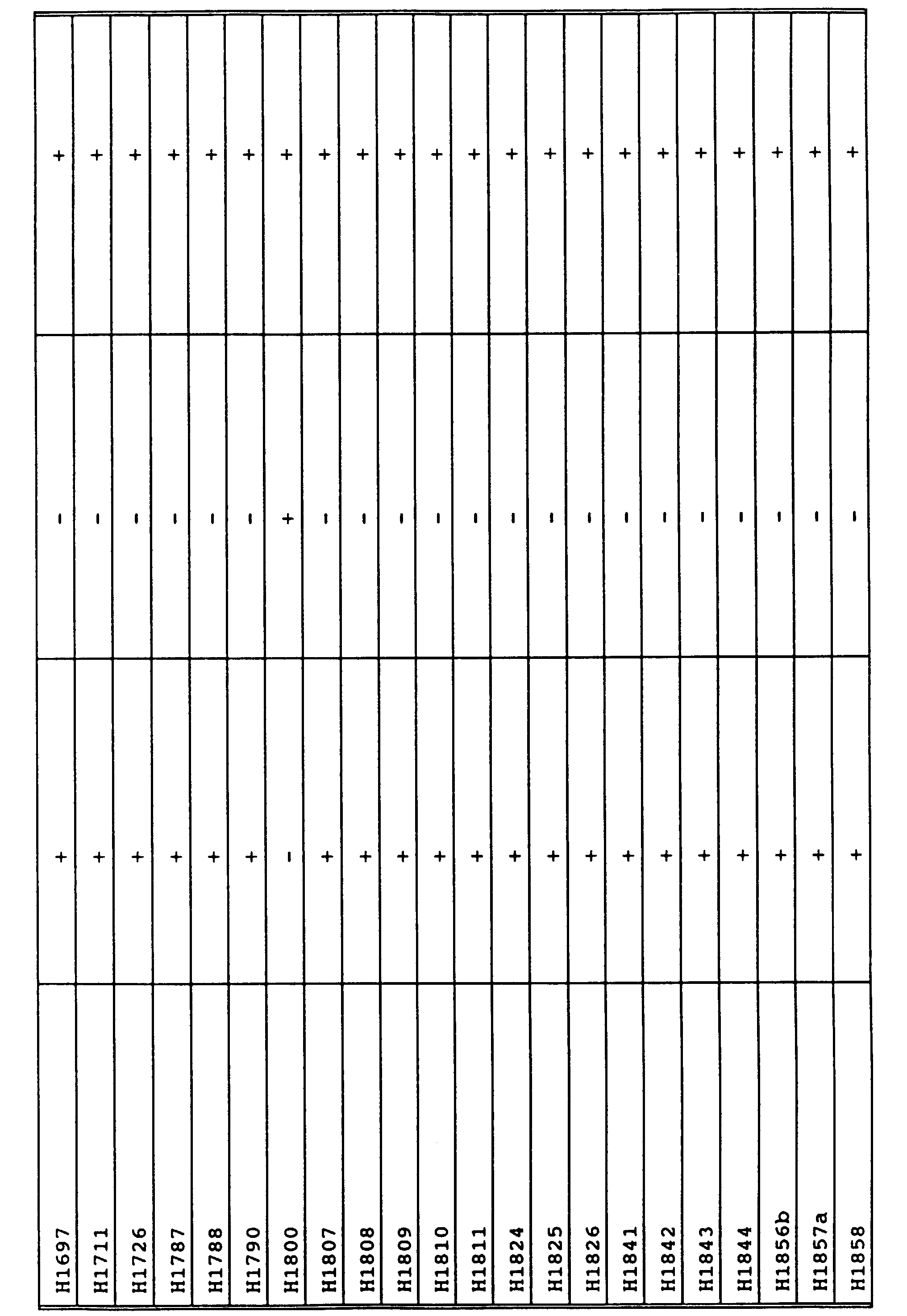

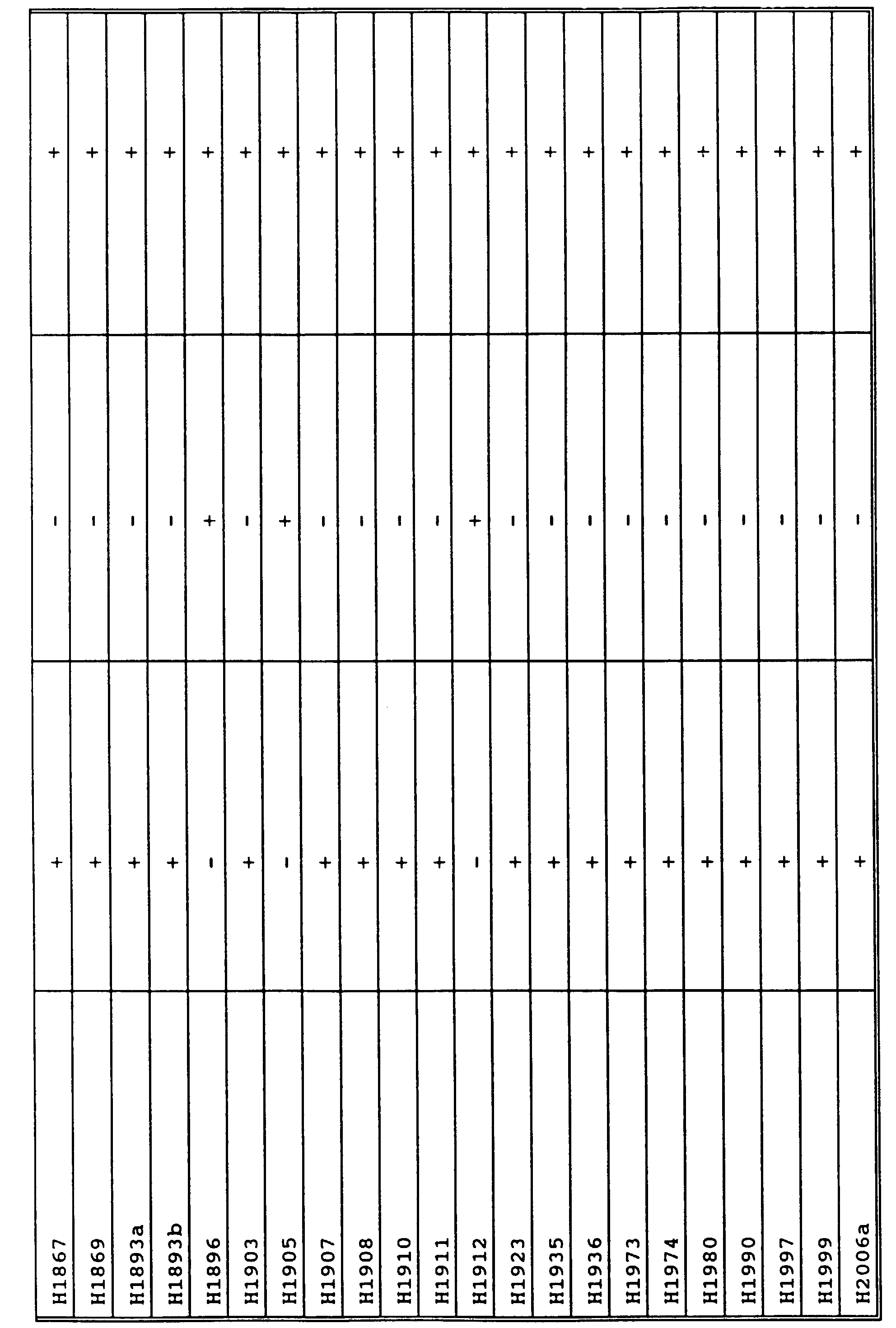

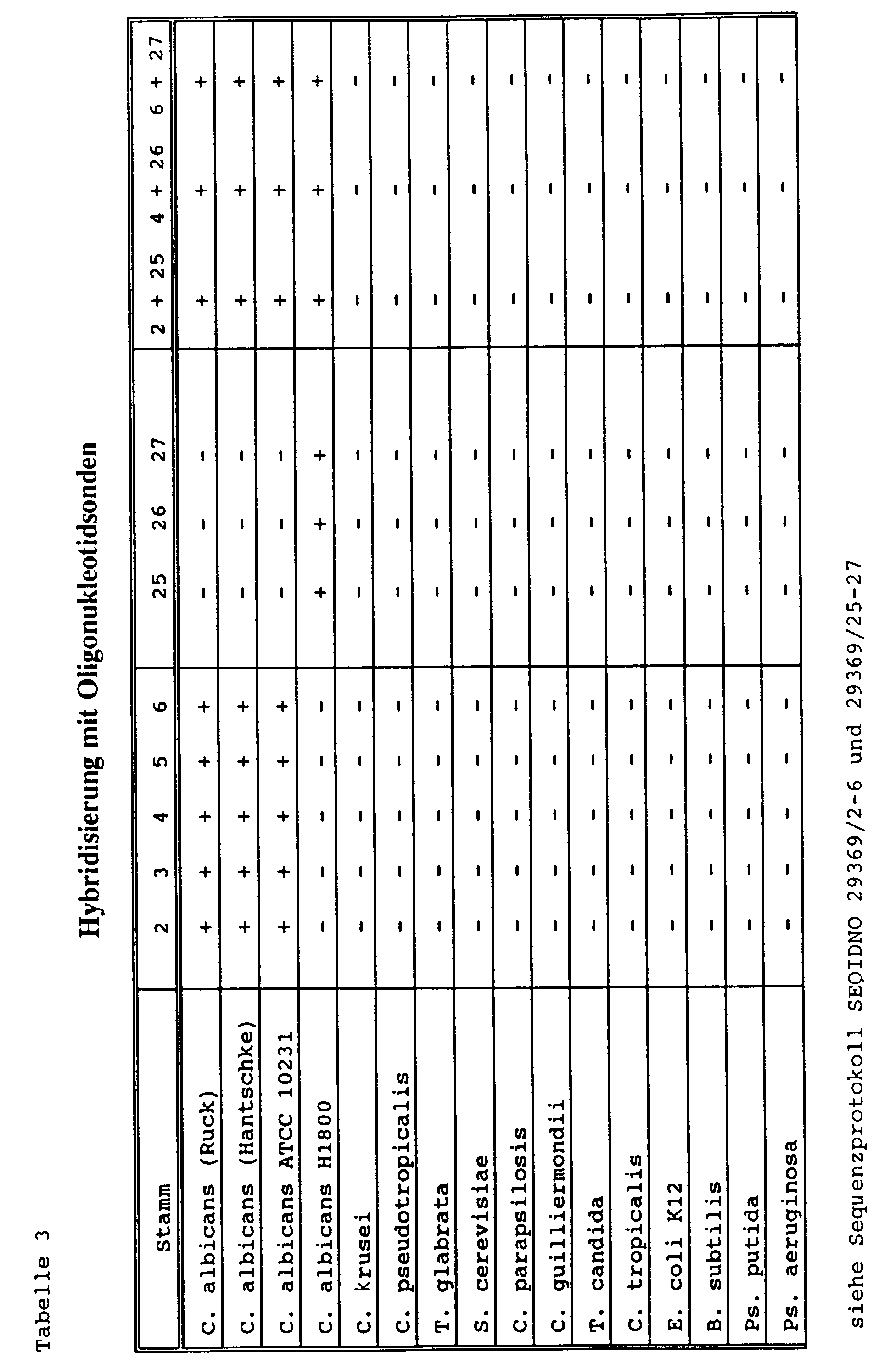

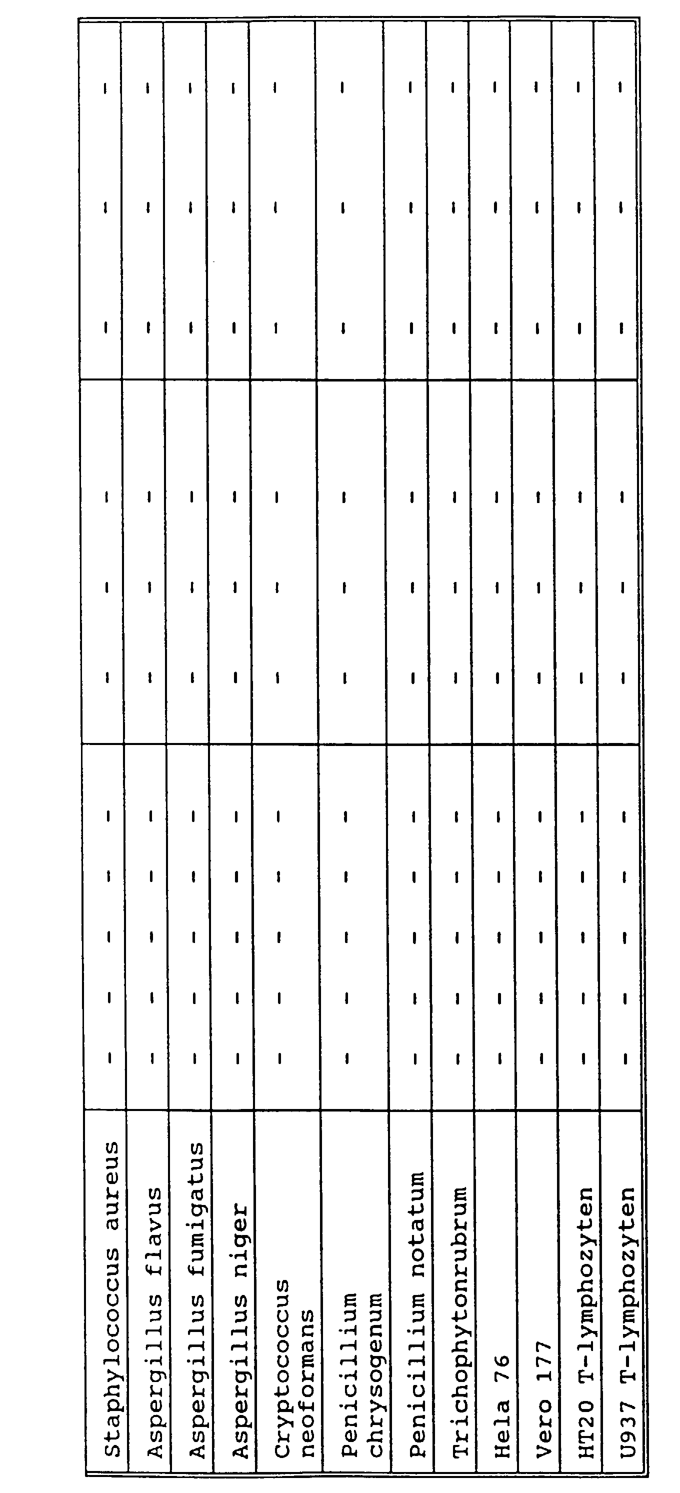

## Patentansprüche

1. Hybridisierungsreagenz, das spezifische mit DNA oder RNA von C. albicans hybridisiert aber nicht mit Nukleinsäuren von anderen Pilzen, Bakterien, Mensch, Tier oder Pflanze, dadurch gekennzeichnet, daß es die folgende DNA oder RNA enthält:
(a) das 7.2 kb BamHI-BamHI C. albicans Fragment oder Teile davon gemäß Fig. 1,
(b) das 2.2 kb ClaI-BamHI C. albicans Fragment oder Teile davon gemäß Fig. 2,
(c) das 2.3 kb ClaI-ClaI C. albicans Fragment oder Teile davon gemäß Fig. 3,
(d) das 6.3 kb BamHI-BamHI C. albicans Fragment oder Teile davon gemäß Fig. 4,
(e) das 2.6 kb ClaI-ClaI C. albicans Fragment oder Teile davon gemäß Fig. 5,
(f) das 3.2 kb ClaI-BamHI C. albicans Fragment oder Teile davon gemäß Fig. 6.

2. Oligonukleotide, die mit DNA oder RNA von C. albicans hybridisieren, aber nicht mit Nukleinsäuren von anderen Pilzen, Bakterien, Mensch, Tier oder Pflanze, dadurch gekennzeichnet, daß sie Nukleotidsequenzen oder Teile davon wie in den Sequenzlistings 2-38 dargestellt enthalten.

## Claims

1. Hybridization reagent which hybridizes specifically with DNA or RNA from C. albicans but not with nucleic acids from other fungi, from bacteria, from humans, from animals or from plants, characterized in that it comprises the following DNA or RNA:
(a) the 7.2 kb BamHI/BamHI C. albicans fragment, or parts thereof, as shown in Fig. 1,
(b) the 2.2 kb ClaI/BamHI C. albicans fragment, or parts thereof, as shown in Fig. 2,
(c) the 2.3 kb ClaI/ClaI C. albicans fragment, or parts thereof, as shown in Fig. 3,
(d) the 6.3 kb BamHI/BamHI C. albicans fragment, or parts thereof, as shown in Fig. 4,
(e) the 2.6 kb ClaI/ClaI C. albicans fragment, or parts thereof, as shown in Fig. 5,
(f) the 3.2 kb ClaI/BamHI C. albicans fragment, or parts thereof, as shown in Fig. 6.

2. Oligonucleotides which hybridize with DNA or RNA from C. albicans but not with nucleic acid from other fungi, from bacteria, from humans, from animals or from plants, characterized in that they contain the nucleotide sequences, or parts thereof, as depicted in sequence listings 2-38.

## Revendications

1. Réactif d'hybridation, qui s'hybride spécifiquement avec l'ADN ou l'ARN de C. albicans mais non avec des acides nucléiques d'autres champignons, de bactéries, de l'homme, d'un animal ou d'une plante, caractérisé en ce qu'il contient l'ADN ou l'ARN suivant :
(a) le fragment Bam-HI-Bam-HI de 7,2 kb de C. albcans ou des parties de ce fragment selon la figure 1,
(b) le fragment ClaI-Bam-HI de 2,2 kb de C. albicans ou des parties de ce fragment selon la figure 2,
(c) le fragment ClaI-ClaI de 2,3 kb de C. albicans ou des parties de ce fragment selon la figure 3,
(d) le fragment Bam-Hi-Bam-HI de 6,3 kb de C. albicans ou des parties de ce fragment selon la figure 4,
(e) le fragment ClaI-ClaI de 2,6 kb de C. albicans ou des parties de ce fragment selon la figure 5,
(f) le fragment ClaI-Bam-HI de 3,2 kb de C. albicans ou des parties de ce fragment selon la figure 6.

2. Oligonucléides, qui s'hybrident avec l'ADN ou l'ARN de C. albicans mais non avec des acides nucléiques d'autres champignons, de bactéries, de l'homme, d'un animal ou d'une plante, caractérisés en ce qu'ils contiennent des séquences ou des parties de séquences de nucléotides telles que représentées dans les listings de séquences 2-38.


### SEQUENZPROTOKOLL

### (1) ALGEMEINE INFORMATION:

(i) ANMELDER:
   (A) NAME: Bayer AG
   (B) STRASSE: Bayerwerk
   (C) ORT: Leverkusen
   (E) LAND: Deutschland
   (F) POSTLEITZAHL: D-51368
   (G) TELEPHON: 0214/3061455
   (H) TELEFAX: 0214/303482
(ii) ANMELDETITEL: Spezifische Gensonden fuer Candida albicans
(iii) ANZAHL DER SEQUENZEN: 44
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: Patent In Release #1.0, Version #1.25 (EPA)
(v) GEGENWÄRTIGE ANMELDEDATEN:
   ANMELDENUMMER: DE 4236708.5

### (2) INFORMATION ZU SEQ ID NO: 1:

(i) SEQUENZ CHARAKTERISTIKA:
   (A) LANGE: 2233 Basenpaare
   (B) ART: Nukleinsäure
   (C) STRANGFORM: Doppel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 1:
   ATCGATGGTA TCGTCGATGG TATCGTCGTT CTGCATGTCG TTGATGTCAA AGCCAATAAA    60
   GTCGTCAAAG TTGTCAAACT TTTGTGGGAC GGCCTCCTCT GCCTTCTTGG CCTCCTCTGC   120
   CTTCTTCTTG GCCTCCTCTG CCTTCTTCTT GGCCTCCTCT GCCTTCTTCT TGGCCTCCTC   180
   TGCCTTCTTC TTGGCCTCCT CTGCCTTCTT CTTGGCCTCC TCTGCCTTCT TCTTGGCCTC   240
   TTCTTCCTTC TTCTTGGCCT CTTCTTCCTT CTTCTTGGCC TCTTCTTCCT TCTTCTTAGC   300
   CTCTTCTTCA CGTTTCTTTC TTGCCGCTGC TTCTATGACT TTCAATTCGT CAAATTTCTT   360
   TTTCTTTTCA ACCATAACGC CGAGACACCA CTCTGCATCA TTGAGTTTCG ACACTGTTTG   420
   TCTAGAATAG CATGGAAGTT TTGGATTTCA CCGTTGAGCA GTTGGGTTAT GGATTTTTGG   480
   ATTAGTTCGT TGTTGGGGCT AGTTATCACA TTGATTTGTT TTCTGTTTTG GTCAATTATC   540
   TCAAATATGT ATCCTGATGA TTTCAATATC TCGTCGAGTG AGTTATGTAA TCTTGTTTGG   600
   AGGTTTTCTG GCATGGTGGG GGGAAATAAA GGTGTGATAA AATTATTCTT GTTGGTTTAT   660
   GCGACGATTG TGTCAGGGAA ATAACTCCTT GATATATGTT AATATATAGG GACATCTACA   720
   CCCAGAGTGT AGACGGAGAC ACGACCTCCG AAGGATCGGT CTGTATAAGG ACCTGGATGT   780
   GACCTAGACG AGCTGGTCAA TCCCGAGGAA TTGACGAAGT ATATATCTAC ATACGCACAC   840
   ATAGTGATTA ATATACTATT AAATGTTCAC TGATACTCAG ATTCCATTAA TATAAGTCGT   900
   ACGTATTAGG TCAACATTGG TGAAGAATTT GCACTCATCA AGAGTCAGGA ATTAGTATAA   960
   AAAGAAGAGA AAACAAAGGT ATTTAGGATA TAGAGACCGA GTTTCAGGAT GGGGGCTATT  1020
   TTTACAACAA AGTGACGTGA AAAGGTCAAT AGCCACGACT TGCAGACTCT TATGCGCAAT  1080
   TATGCAAGTT GTTGCCTTTT GTTTACATTT TTTTGTTTAC ATTTTTTGTC GACCTAAGCA  1140
   ACATTCATCC CTTGCCCGGC CCCCTATTTT CACCACCATA AATGCCATGC TGGCGTGACC  1200
   TCAAAAATTT TACTATCCCT ATCACTTTCT TTTCCTTCTT TTTTCACCAT TCGTTCATCA  1260
   ATCCATTAGT TTTCATCATA GATCGACTGA ATAAGTGCTA CTACTTCGAC TACTGATCCT  1320
   TTAAGTTATG GCAAGTATGA ATAATACAAC TTTTTTGAAG AATTTTGTTT ACTAACTAAG  1380
   TATTATAGAA AAGTCAGAGA TTATTGTTCA TGATTGTATC GGTTTTCATC ATAAATCATT  1440
   GAAAAGATTC TAGTCAAGTG TTTCAGAGAT GCCCTAAGTT AAGATATGTA TGATCATTAC  1500
   CTATGGTTTT GAAGAATTTT GTTTACTAAC TAACTAATAT AGATAATTTA TTGATTATTG  1560
   TTTGGAGACT ACCCATAATG CTTCTGGTAT CGCGGTTCGG TCAGCTATAA TGATTACACA  1620
   ATAGGGTACT TCTATTTGAC AACTTCTGTC ACATGTTATT GCTTGATTTG GCTTGTTCTA  1680
   AAGTGTTTTT TTTTACTGGA AATTGATTAT TCAGGGGTTT GTAATTGACC ATAAAATCTA  1740
   CATTAAAGTT GGCCAAATGG ACTATCTGAC TTGCTTTGAT AAGAGATGAC AGGATTTTAA  1800
   GGATATCAAC CTAGTTTTCG GTGAATTTAT GGTATCAATT GAACTTGTGG TCCGTGACAA  1860
   GGATTACTGA TAGATAAACC GTTTTTGCAA GGTACATTTA CCAGTCAACT AGGATTTATG  1920
   ATTCAATATC AAAAACAGAA ACCAATTATC ACAATGTAGG TTCTAATATA TAGTGGTTGT  1980
   GTATACACCA AGAAGTTAAT GATTTGTTCA TAATTAGTGT GACAGACTAC ATGAAGTATA  2040
   CTATGACTTC TCTTGATCAG TGAAACGCGC GATTGTTATT GATTTTAATA TAAAAACGCC  2100
   ACTTTAAATA CATTTAAAAA AATGTAAACA AAACTTTTAA AACAGTTGAA ACACTGAAAT  2160
   CAATATAACC AATGCCTAAC ACCAACGAAT TTGAAGACTC ATCTCAGCAA TCTAGAGACA  2220
   AACTTTTGGA TCC                                                     2233

### (2) INFORMATION ZU SEQ ID NO: 2:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 2:
   ATCGATGGTA TCGTCGATGG TATCGTCGTT CTGCATGTCG TTGATGTCAA AGCCAATAAA    60
   GTCGTCAAAG TTGTCAAACT TTTGTGGGAC GGCCTCCTCT                          100

### (2) INFORMATION ZU SEQ ID NO: 3:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 3:
   GCCTTCTTGG CCTCCTCTGC CTTCTTCTTG GCCTCCTCTG CCTTCTTCTT GGCCTCCTCT    60
   GCCTTCTTCT TGGCCTCCTC TGCCTTCTTC TTGGCCTCCT                          100

### (2) INFORMATION ZU SEQ ID NO: 4:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 4:
   CTGCCTTCTT CTTGGCCTCC TCTGCCTTCT TCTTGGCCTC TTCTTCCTTC TTCTTGGCCT 60
   CTTCTTCCTT CTTCTTGGCC TCTTCTTCCT TCTTCTTAGC 100

### (2) INFORMATION ZU SEQ ID NO: 5:

(i) SEQUENZ CHARAKTERISTIKA:
   (A) LANGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 5:
   CTCTTCTTCA CGTTTCTTTC TTGCCGCTGC TTCTATGACT TTCAATTCGT CAAATTTCTT 60
   TTTCTTTTCA ACCATAACGC CGAGACACCA CTCTGCATCA 100

### (2) INFORMATION ZU SEQ ID NO: 6:

(i) SEQUENZ CHARAKTERISTIKA:
   (A) LANGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 6:
   TTGAGTTTCG ACACTGTTTG TCTAGAATAG CATGGAAGTT TTGGATTTCA CCGTTGAGCA 60
   GTTGGGTTAT GGATTTTTGG ATTAGTTCGT TGTTGGGGCT 100

### (2) INFORMATION ZU SEQ ID NO: 7:

(i) SEQUENZ CHARAKTERISTIKA:
   (A) LANGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 7:
   AGTTATCACA TTGATTTGTT TTCTGTTTTG GTCAATTATC TCAAATATGT ATCCTGATGA 60
   TTTCAATATC TCGTCGAGTG AGTTATGTAA TCTTGTTTGG 100

### (2) INFORMATION ZU SEQ ID NO: 8:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 8:
   AGGTTTTCTG GCATGGTGGG GGGAAATAAA GGTGTGATAA AATTATTCTT GTTGGTTTAT 60
   GCGACGATTG TGTCAGGGAA ATAACTCCTT GATATATGTT 100

### (2) INFORMATION ZU SEQ ID NO: 9:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 9:
   AATATATAGG GACATCTACA CCCAGAGTGT AGACGGAGAC ACGACCTCCG AAGGATCGGT    60
   CTGTATAAGG ACCTGGATGT GACCTAGACG AGCTGGTCAA                         100

### (2) INFORMATION ZU SEQ ID NO: 10:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 10:
   TCCCGAGGAA TTGACGAAGT ATATATCTAC ATACGCACAC ATAGTGATTA ATATACTATT    60
   AAATGTTCAC TGATACTCAG ATTCCATTAA TATAAGTCGT                         100

### (2) INFORMATION ZU SEQ ID NO: 11:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 11:
   ACGTATTAGG TCAACATTGG TGAAGAATTT GCACTCATCA AGAGTCAGGA ATTAGTATAA    60
   AAAGAAGAGA AAACAAAGGT ATTTAGGATA TAGAGACCGA                         100

### (2) INFORMATION ZU SEQ ID NO: 12:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 12:
   GTTTCAGGAT GGGGGCTATT TTTACAACAA AGTGACGTGA AAAGGTCAAT AGCCACGACT    60
   TGCAGACTCT TATGCGCAAT TATGCAAGTT GTTGCCTTTT                         100

### (2) INFORMATION ZU SEQ ID NO: 13:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICRE HERKUNFT:
   (A) ORGANISMUS: Candida albicans
(xi) SEQUENZBESCHREIBUNG: SEQ ID NO: 13:
   GTTTACATTT TTTTGTTTAC ATTTTTTGTC GACCTAAGCA ACATTCATCC CTTGCCCGGC 60
   CCCCTATTTT CACCACCATA AATGCCATGC TGGCGTGACC 100

### (2) INFORMATION ZU SEQ ID NO: 14:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 14:
   TCAAAAATTT TACTATCCCT ATCACTTTCT TTTCCTTCTT TTTTCACCAT TCGTTCATCA 60
   ATCCATTAGT TTTCATCATA GATCGACTGA ATAAGTGCTA 100

### (2) INFORMATION ZU SEQ ID NO: 15:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 15:
   CTACTTCGAC TACTGATCCT TTAAGTTATG GCAAGTATGA ATAATACAAC TTTTTTGAAG 60
   AATTTTGTTT ACTAACTAAG TATTATAGAA AAGTCAGAGA 100

### (2) INFORMATION ZU SEQ ID NO: 16:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 16:
   TTATTGTTCA TGATTGTATC GGTTTTCATC ATAAATCATT GAAAAGATTC TAGTCAAGTG   60
   TTTCAGAGAT GCCCTAAGTT AAGATATGTA TGATCATTAC                        100

### (2) INFORMATION ZU SEQ ID NO: 17:

(i) SEQUENZ CHARAKTERISTIKA:
   (A) LANGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 17:
   CTATGGTTTT GAAGAATTTT GTTTACTAAC TAACTAATAT AGATAATTTA TTGATTATTG   60
   TTTGGAGACT ACCCATAATG CTTCTGGTAT CGCGGTTCGG                        100

### (2) INFORMATION ZU SEQ ID NO: 18:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 18:
   TCAGCTATAA TGATTACACA ATAGGGTACT TCTATTTGAC AACTTCTGTC ACATGTTATT 60
   GCTTGATTTG GCTTGTTCTA AAGTGTTTTT TTTTACTGGA 100

### (2) INFORMATION ZU SEQ ID NO: 19:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 19:
   AATTGATTAT TCAGGGGTTT GTAATTGACC ATAAAATCTA ACATTAAAGT GGCCAAATGG 60
   ACTATCTGAC TTGCTTTGAT AAGAGATGAC AGGATTTTAA 100

### (2) INFORMATION ZU SEQ ID NO: 20:

(i) SEQUENZ CHARAKTERISTIKA:
   (A) LANGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 20:
   GGATATCAAC CTAGTTTTCG GTGAATTTAT GGTATCAATT GAACTTGTGG TCCGTGACAA    60
   GGATTACTGA TAGATAAACC GTTTTTGCAA GGTACATTTA                         100

### (2) INFORMATION ZU SEQ ID NO: 21:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 21:
   CCAGTCAACT AGGATTTATG ATTCAATATC AAAAACAGAA ACCAATTATC ACAATGTAGG    60
   TTCTAATATA TAGTGGTTGT GTATACACCA AGAAGTTAAT                         100

### (2) INFORMATION ZU SEQ ID NO: 22:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 22:
   GATTTGTTCA TAATTAGTGT GACAGACTAC ATGAAGTATA CTATGACTTC TCTTGATCAG     60
   TGAAACGCGC GATTGTTATT GATTTTAATA TAAAAACGCC                         100

### (2) INFORMATION ZU SEQ ID NO: 23:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 23:
   ACTTTAAATA CATTTAAAAA AATGTAAACA AAACTTTTAA AACAGTTGAA ACACTGAAAT        60
   CAATATAACC AATGCCTAAC ACCAACGAAT TTGAAGACTC                             100

### (2) INFORMATION ZU SEQ ID NO: 24:

(i) SEQUENZ CHARAKTERISTIKA:
   (A) LÄNGE: 33 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 24:
   ATCTCAGCAA TCTAGAGACA AACTTTTGGA TCC                                     33

### (2) INFORMATION ZU SEQ ID NO: 25:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 25:
   ATCGATTGAT TTTCAATTTC TTATTGTTTG AATGATGTGG CTCAACAAAA TGAAAATGGA     60
   GAAACACCAT AAAACACGGT AGAAGTTGCC AATTTTGTTT                           100

### (2) INFORMATION ZU SEQ ID NO: 26:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 26:
   AAGAATAAAT CCACCAATAT ATATACACAA AGTTCTTACG AATGTTTGTG TTATGATTTT     60
   GAGCCCACAA GGACTTTGCA GAGGATTATT GTTTAATTGG                           100

### (2) INFORMATION ZU SEQ ID NO: 27:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 27:
   TTGGCTTGTA TTCTATGTTG GAACTGACTT TTTTTTACTT TGCTTCTCTT TATTTGGAAA 60
   CTCCATATAC CTCGTTGGAA TATATAAGAA GTACTAATAT 100

### (2) INFORMATION ZU SEQ ID NO: 28:

(i) SEQUENZ CHARAKTERISTIKA:
   (A) LANGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 28:
   GTCACTAGAA AGTATTATAT TACAACTTGG AATGTATAAT AATATCTTCA AGCAATTCAT 60
   ATCAACAACA ATTCTTTTGC TATCAGCATC ACTGCACCAA 100

### (2) INFORMATION ZU SEQ ID NO: 29:

(i) SEQUENZ CHARACTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 29:
   AAAAACAGCC TTGATAATAA CAATAATGCA GATGATGATA AATATGCAAG AATAAAAGTT 60
   AGTCAATTCT GGTTATTAAC GTGAATGAAT GAATAGATGA 100

### (2) INFORMATION ZU SEQ ID NO: 30:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 30:
   AATCTGCAAT CGTAAGAGTG TAGGTGAAGG TGAAATGCAT AAAAAATTAA CAAATCTGAA      60
   CAATCAACAC GCCTTTGACC TCTTTTTTTT TTTATTATTT                           100

### (2) INFORMATION ZU SEQ ID NO: 31:

(i) SEQUENZ CHARAKTERISTIKA:
   (A) LÄNGE: 22 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 31:
   CTTGCAACGT TCTTTTCTAA TT                                              22

### (2) INFORMATION ZU SEQ ID NO: 32:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 32:
   TGAACCTGTT GGATTAGTTT GTGTTGTAGT AGTAGTGATT GTTCCTGTCC ATTCACTAGT    60
   AACAGTTGTG GTAGTGTGAT ATGGAACATC AACAATAACA                         100

### (2) INFORMATION ZU SEQ ID NO: 33:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 33:
   GTAGCAGTTT GTCCTATTGG TACAGTTTGG GTTCTGTAGG AAGTAGTCAC ACCAACATAT    60
   GATGTTGTGA TGGTAGTTGT TGGAATGGGT TGCAAAATTT                         100

### (2) INFORMATION ZU SEQ ID NO: 34:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 34:
   CAATTGTTTT AGTTTTGTCA ACATCGGAAT TGAATGGTAA AGTAGTCACA GCAGTAGTAC    60
   TATCTGTAAC TGTTCTAGTT GTAGCCACAA TGACAATACC                         100

### (2) INFORMATION ZU SEQ ID NO: 35:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 35:
   CTTGGAACCA GCTTCACTAT TATTGTATCC GGACCATCTC AAAGTAAATG GATCATCTAT 60
   AAAACTGCTA CCAACACAAG TATAATTATT AGCATATGAC 100

### (2) INFORMATION ZU SEQ ID NO: 36:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 36:
   AAAATATACT GTTTAACATC CGGAGCAGAT ACATAAACGT CAATAAATGG ACGATAACCT 60
   GCAGGGATAT TTTCATATGT GATAGAGATA CCATTAGATG 100

### (2) INFORMATION ZU SEQ ID NO: 37:

(i) SEQUENZ CHARAKTERISTIKA:
   (A) LÄNGE: 100 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 37:
   AACAATTTTT CTTGTAACTA AATGAATCAT ATGATAACGG ATGATCCCAA TCATTCACTC    60
   CTTTGAAATC CCAATATGAA CATTTGAACA GTCTATAGTG                          100

### (2) INFORMATION ZU SEQ ID NO: 38:

(i) SEQUENZ CHARAKTERISTIKA:
   (A) LANGE: 99 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: NEIN
(vi) URSPRÜNLICHE HERKUNFT:
   (A) ORGANISMUS: Candida albicans
(xi) SEQUENZBESCHREIBUNG: SEQ ID NO: 38:
   TATGCGCACC CGTTCTCGGA GCACTGTCCG ACCGCTTTGG CCGCCGCCCA GTCCTGCTTT    60
   CGCTACTTGG AGCTCAACTA TCGACTACGC GCTCATGTG                           99
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: JA
(iii) ANTISENSE: NEIN
(vi) URSPRÜNLICHE HERKUNFT:
   (C) INDIVIDUUM ISOLAT: primer-DNA
(xi) SEQUENZBESCHREIBUNG: SEQ ID NO: 39:
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: JA
(iii) ANTISENSE: NEIN
(vi) URSPRÜNLICHE HERKUNFT:
   (C) INDIVIDUUM ISOLAT: primer-DNA
(xi) SEQUENZBESCHREIBUNG: SEQ ID NO: 40:
   (B) ART; Nukleinsaure
   (C) STRANGFORM: Einzel
   (D) TOPOLOGIE: linear
(ii) ART DES MOLEKÜLS: DNS (genomisch)
(iii) HYPOTHETISCH: JA
(iii) ANTISENSE: NEIN
(vi) URSPRÜNLICHE HERKUNFT:
   (C) INDIVIDUUM ISOLAT: primer-DNA
(xi) SEQUENZBESCHREIBUNG: SEQ ID NO: 41:
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: JA
(iii) ANTISENSE: NEIN
(vi) URSPRÜNLICHE HERKUNFT:
   (C) INDIVIDUUM ISOLAT: primer-DNA
(xi) SEQUENZBESCHREIBUNG: SEQ ID NO: 42:
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: JA
(iii) ANTISENSE: NEIN
(vi) URSPRÜNLICHE HERKUNFT:
   (C) INDIVIDUUM ISOLAT: primer-DNA
(xi) SEQUENZBESCHREIBUNG: SEQ ID NO: 43:
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: JA
(iii) ANTISENSE: NEIN
(vi) URSPRÜNLICHE HERKUNFT:
   (C) INDIVIDUUM ISOLAT: primer-DNA
(xi) SEQUENZBESCHREIBUNG : SEQ ID No: 44: